(19) Europäisches Patentamt
European Patent Office
Office européen des brevets

(11) **EP 2 586 782 B2**

(12) **NEW EUROPEAN PATENT SPECIFICATION**
After opposition procedure

(45) Date of publication and mention of the opposition decision:
**04.10.2017 Bulletin 2017/40**

(45) Mention of the grant of the patent:
**29.10.2014 Bulletin 2014/44**

(21) Application number: **12193696.7**

(22) Date of filing: **02.07.2009**

(51) Int Cl.:
*C07D 487/04* (2006.01) *A61K 31/155* (2006.01)

(54) **Crystalline salts of sitagliptin**
Kristalline Salze von Sitagliptin
Sels cristallins de sitagliptine

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.07.2008 IN CH16312008**

(43) Date of publication of application:
**01.05.2013 Bulletin 2013/18**

(60) Divisional application:
**14190672.7 / 2 915 814**

(62) Document number(s) of the earlier application(s) in accordance with Art. 76 EPC:
**09772169.0 / 2 318 411**

(73) Proprietor: **ratiopharm GmbH**
**89079 Ulm (DE)**


(72) Inventors:
- **Gidwani, Matioram Ramesh**
**400 080 Mumbai, Maharashtra (IN)**
- **Hiremath, Channaveerayya**
**400 082 Mumbai, Maharashtra (IN)**


(74) Representative: **Greiner, Elisabeth**
**df-mp Dörries Frank-Molnia & Pohlman**
**Patentanwälte Rechtsanwälte PartG mbB**
**Theatinerstraße 16**
**80333 München (DE)**


(56) References cited:
**WO-A-2005/072530**
**WO-A2-2004/087650**
**WO-A2-2007/035198**
**WO-A2-2009/085990**
**WO-A2-2010/012781**
**WO-A2-2010/117738**
- **IN 3076/CHE/2007**
- **IN 159/CHE/2008**
- **IN 1188/CHE/2008**
- **US 61/058,764**
- **US 61/058,975**
- **US 61/097,910**
- **AHRÉN B.: 'Novel combination treatment of type 2 diabetes DPP-4 inhibition + metformin' VASCULAR HEALTH AND RISK MANAGEMENT vol. 4, no. 2, April 2008, pages 383 - 394**
- **DAVIES G.: 'Changing the salt, changing the drug' THE PHARMACEUTICAL JOURNAL vol. 266, 10 March 2001, pages 322 - 323**
- **GENNARO A.: 'Remington's Pharmaceutical Sciences', vol. 18TH ED., 1990, MACK PUBLISHING COMPANY, EASTON page 1444**
- **FDA Leaflet Information "Januvia" of 2006**
- **USP 29. Physical Tests, Chapter <711> Dissolution, 2006, pages 2675-2682**
- **STAHL P.: 'Handbook of pharmaceutical salts', 2011, VERLAG HELVETICA CHIMICA ACTA, ZÜRICH pages 345 - 377**
- **BERNSTEIN J.: 'Polymorphism in Molecular Crystal', 2002, CLARENDON PRESS, OXFORD pages 111 - 125**
- **HORGAN D.E. ET AL: 'Advanced topics in crystallisation', vol. CHAPT.3, 2015, INTECH pages 57 - 84**
- **MURACHANIAN D.: 'Two-piece hard capsules for pharmaceutical formulations' PHARMACEUTICAL PROCESSES vol. 14, no. 3, 2010, pages 31 - 42**
- **European pharmacopeia 5.0, 2004, 599-600**


EP 2 586 782 B2

Remarks:

The file contains technical information submitted after the application was filed and not included in this specification

## Description

The present invention relates to crystalline salt of the dipeptidyl peptidase-IV inhibitor sitagliptin with malic acid.

## Background of the invention

The enzyme dipeptidyl peptidase-IV (DPP-IV) is responsible for the degradation of incretins such as the glucagon-like peptide-I (GLP-I) and the gastric inhibitory polypeptide (GIP), also known as the glucose-dependent insulinotropic peptide.

The inhibition of the enzyme DPP-IV is a new approach in the therapy of Type 2 Diabetes, alto known as non-insulin dependent diabetes mellitus (NIDDM).

The new therapeutic approach has been described previously in scientific literature: C. F. Deacon and J. J. Holst, "Dipeptidyl peptidase IV inhibition as an approach to the treatment and prevention of Type 2 diabetes: a historical perspective", Biochem. Biophys. Res. Commun., 294 (2000) 1-4; K. Augustyns, et al., "Dipeptidyl peptidase IV inhibitors as new therapeutic agents for the treatment of Type 2 diabetes, "Expert. Opin. Ther. Patents, 13 (2003) 499-510; D. J. Drucker, "Therapeutic potential of dipeptidyl peptidase IV inhibitors for the treatment of Type 2 diabetes", Expert Opin. Investig. Drugs, 12 (2003) 87-100, and M. A. Nauck et al., "Incretins and Their Analogues as New Antidiabetic Drugs, "Drug News Perspect., 16 (2003) 413-422.

EP 0 896 538 describes the use of a DPP-IV inhibitor to lower the blood glucose level in mammals.

WO 2003/004498 teaches β-amino-tetrahydroimidazo-(1,2a)-pyrazine and β-amino-tetrahydroimidazolo-(4,3a)-pyrazine as dipeptidyl peptidase inhibitors for the therapy and prevention of diabetes. One of these pyrazines is sitagliptin (7-[(3*R*)-3-amino-1-oxo-4-(2,4,5-trifluorophenyl)butyl]-5,6,7,8-tetrahydro-3-(trifluoromethyl)-1,2,4-triazolo[4,3-*a*]pyrazine) having the following structural formula:

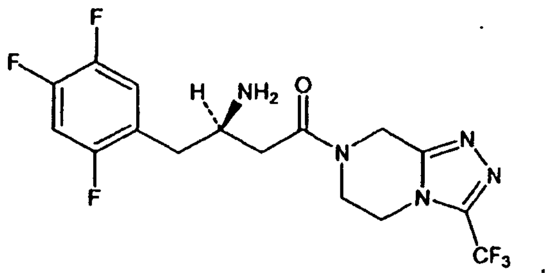

A list of pharmaceutical acceptable salts is generally included in WO 03/004498. The crystalline salts of the present invention are not disclosed therein.

WO 2005/072530 discloses specific crystalline salts of sitagliptin and hydrochloric acid, benzene sulfonic acid, p-touluene sulfonic acid, *D*- and *L*-tartaric acid and (1*S*)-(+)- and (1*R*)-(-)-10-camphorsulfonic acid.

D. Kim et al. mention in J. Med. Chem. 48 (2005) 141-151 that sitagliptin fumarate, having a 2:1 stoichiometry, was used for *in-vivo* studies.

WO 2005/003135 discloses sitagliptin dihydrogenphosphate and its crystalline monohydrate. Four crystalline polymorphs of sitagliptin dihydrogenphosphate anhydrate are disclosed in WO 2005/020920 and WO 2005/030127. Amorphous sitagliptin dihydrogenphosphate is claimed in WO 2006/033848.

Once formulated to solid dosage forms, the prior art salts of sitagliptin are of limited stability, hence a certain degree of decomposition can be observed. Furthermore, many salts exhibit an undesired hygroscopicity.

In the pharmaceutical industry there is a need for stable salts of sitagliptin, which exhibit good stability and may be formulated even after prolonged storage times. In solid dosage forms the salts should be stable, preferably they should show a higher stability than the salts of the prior art and lower hygroscopicity.

It is therefore an object of the present invention to provide sitagliptin in a form having a good chemical and/or physical stability, a low hygroscopicity, a good solubility, a good bioavailability and/or has a good processability, both during its preparation and in the preparation of pharmaceutical compositions containing the sitagliptin.

It has now been found that one or more of the above problems can be solved by providing sitagliptin in the form of a crystalline salt with malic acid. Thus, the present invention relates to a crystalline salt of sitagliptin with malic acid having in the XRPD pattern (determined by using Cu K radiation) characteristic peaks at 2 theta angles of 15.9 $\pm$ 0.2°, 18.0 $\pm$ 2° and 23.2 $\pm$ 0.2°

Further crystalline salts of sitagliptine with monobasic, dibasic or tribasic acids are described herein.

The crystalline salt of the present invention can be solvent free or can contain solvent molecules in its crystal structure. Thus, the present invention also covers any hydrate or anhydrate or solvate form of the crystalline salt of

sitagliptin.

The acid in the crystalline salts described herein may, for example, be selected from HCl, $H_2SO_4$, $H_3PO_4$, sulfonic acids, such as methanesulfonic acid, as well as mono, di and tricarboxylic acids. In one embodiment the acid is not $H_3PO_4$.

If the acid is a carboxylic acid, it can be of the general structural formula $R^1$-COOH wherein $R^1$ is hydrogen, carboxyl, $C_{1-4}$ alkyl or $C_{2-4}$ alkenyl, wherein the $C_{1-4}$ alkyl and the $C_{2-4}$ alkenyl may optionally be substituted with 1-2 carboxyl, 1-3 hydroxyl, 1-3 amino, 1-3 phenyl and/or 1-3 $C_{1-5}$ alkyl.

Any of the above alkyl and alkenyl groups may be linear or branched or, if it contains at least three carbon atoms, may form a ring. The alkyl groups may, for example, be methyl, ethyl, propyl, n-butyl, tert-butyl and pentyl.

Preferred acids of the above general structural formula $R^1$-COOH are those, wherein $R^1$ is $C_2$ alkyl or $C_2$ alkenyl each of which is substituted with 1 carboxyl and optionally with 1 hydroxyl or amino. Tartaric acid is preferably not employed.

Examples of the above described carboxylic acids are fumaric acid, malonic acid, malic acid, succinic acid, lactic acid, glycolic acid, maleic acid, citric acid, aspartic acid and mandelic acid.

Examples for a dicarboxylic acids are succinic acid, malonic and fumaric acid, D- and L-tartaric acid, D- and L-malic acid and L-aspartic acid. One example for a tribasic acid is citric acid. Examples of monocarboxylic acids are glycolic acid and lactic acid.

Yet another embodiment of the present invention concerns a sitagliptin malate in which sitagliptin and the acid are substantially in a 1:1 acid to base stoichiometry wherein the malic acid is only singly deprotonated.

Herein, a novel form of sitagliptin hydrochloride is described. The new polymorph of sitagliptin hydrochloride is characterised by DSC and XRD. DSC shows onset of melting transition point at 195.8 °C ± 2 °C, and a peak melting point at 202.3 °C ± 2 °C as measured by DSC. Further sitagliptin hydrochloride salt described herein shows a pH of 5.8 ± 0.1 in 2 % aqueous solution. Further, the novel form of sitagliptin hydrochloride salt is not hygroscopic even under prolonged storage at 93 % relative humidity. Further, sitagliptin hydrochloride salt described herein shows excellent stability when stored at 60 °C for 4 weeks.

Furthermore, sitagliptin hemisulfate is described. The ratio of sitagliptin to sulfuric acid is 2:1. Sitagliptin hemisulfate form II is characterised by DSC and XRD. DSC shows onset of melting transition at 169.3 °C ± 2 °C, and a peak melting point at 175.9 °C ± 2 °C as measured by DSC. Further, sitagliptin hemisulphate salt described herein shows excellent stability when stored at 60 °C for 4 weeks. The sulfate salt shows very high solubility in water as compared to other known salts of sitagliptin.

Specific polymorphs or crystalline sitagliptin malate of the present invention are described in the examples. Further crystalline salts of sitagliptin are also described in the examples. These polymorphs are characterized by their DSC data and/or their XRPD data. In one embodiment these polymorphs are characterized by three characteristic peaks in the XRPD pattern at the 2 theta angles given in the claims for each of the polymorphs. In a further embodiment these polymorphs are characterized by the 2 theta angles of the five peaks in the XRPD pattern having the highest intensities. Most preferably, the polymorphs are characterized by the XRPD patterns as shown in the figures.

Another embodiment of the present invention is a pharmaceutical formulation comprising one or more salts of the present invention and a pharmaceutically acceptable carrier or diluent.

Another embodiment of the present invention is a pharmaceutical formulation comprising a combination of one or more salts of the present invention and one or more active pharmaceutical ingredients for the simultaneous, separate or sequential use in the therapy. Examples for active pharmaceutical ingredients are anti-diabetics, e.g., metformin, pioglitazone or rosiglitazone.

Yet another embodiment of the present invention is sitagliptin malate for use in the treatment or prevention of non insulin dependent diabetes mellitus, obesity, insulin resistance, syndrome X and type 2 diabetes.

Another aspect of the present invention is a process for the preparation of a sitagliptin salt of the present invention comprising the steps of

i) formation of a solution or dispersion comprising sitagliptin and a solvent, optionally heating the solution,

ii) addition of an acid,

iii) inducing the crystallization of the sitagliptin salt, optionally by addition of seed crystals or of an anti-solvent or by cooling the solution,

iv) recovering of the crystals, and optionally

v) drying of the crystals.

The sitagliptin salts of the present invention, and their solvates, hydrates and polymorphic forms, exhibit improved properties compared to the free base. The sitagliptin salts of the present invention are more stable and of improved quality compared to the free base, which is advantageous for the storage of raw material as well as for the distribution of the final product.

Furthermore, the salts of the present invention exhibit a higher solubility in aqueous solution, in particular under physiological conditions, compared to the free base or the phosphate salt. These properties are advantageous because the dissolution is faster and a smaller amount of water is needed for complete solvation. This may lead to a higher bioavailability compared to the free base, especially in the case of solid dosage forms.

Compared to sitagliptin salts of prior art, the novel sitagliptin salts exhibit a lower hygroscopicity. This leads to an improved stability due to reduced decomposition caused by hydrolysis. In the pharmaceutical sector there is a constant need for sitagliptin salts with improved stability, which can be further processed to a pharmaceutical formulation. In solid pharmaceutical formulations these salts shall be at least as stable as salts of the prior art and show a low hygroscopicity.

Furthermore, the sitagliptin salts of the present invention exhibit a lower tendency to decomposition by hydrolysis. Typically, an aqueous solution of 0.020 kg sitagliptin in 1 litre of water shows a pH of 4-7, preferably 4.5-6.0.

The sitagliptin salts of the present invention have an improved solubility and are preferentially suitable for direct compression. Due to their superior crystallinity the salts of the present invention are especially suitable for the preparation of tablets with good solubility.

The sitagliptin salts of the present invention have very high chiral purity wherein the chiral purity is typically between 99 % and 100 %.

The sitagliptin salts of the present invention can be prepared from the free base by methods known to those skilled in the art.

Generally, salts can be prepared by reaction of the free base with the respective acid in water or suitable organic solvents or in a mixture of water and one or more suitable organic solvents.

Furthermore, the sitagliptin salts of the present invention can be prepared by reacting a sitagliptin salt of a first acid with a second acid, provided that the second acid is stronger and sets free the first, weaker acid.

Examples for non-aqueous solvents are ethers, preferably diethyl ether, esters, preferably ethyl acetate, alcohols, preferably ethanol and iso-propanol, and acetonitrile. Preferred organic solvents are those which are at least partially miscible with water. Examples for such solvents are alcohols, for example methanol, ethanol, n-propanol, iso-propanol, and butanol, ketones, preferably acetone or methyl ethyl ketone, acetonitrile, dimethyl formamide (DMF), and dimethyl sulfoxide (DMSO).

In case of mixtures of water and organic solvents the proportion of organic solvents is between 10% and 90%, preferably between 20% and 70%, most preferably between 30% and 50%. In case of higher alcohols, which are less polar solvents and only partially water miscible, the percentage of the organic solvent can be lower.

In a preferred embodiment the crystallization is induced by addition of at least one seed crystal. In order to achieve a quantitative crystallization the solution can be cooled.

Yet another embodiment of the invention comprises the use of an anti-solvent. By adding an anti-solvent the solubility of a salt in a certain solvent is reduced. Examples for anti-solvents are $C_{3-7}$ alkyl nitriles, in particular acetonitrile, esters, $C_{2-7}$ alkyl carbonic acid $C_{1-5}$ alkyl esters, in particular acetic acid ethyl ester or acetic acid isopropyl ester, di-($C_1$-$C_5$-alkyl)-ether, for example methyl tert.-butyl ether (MTBE), and tetrahydrofurane (THF), $C_5$-$C_8$-alkanes, in particular pentane, hexane or heptane. MTBE is especially preferred.

The salts may also have the advantage that they are more efficacious, less toxic, longer acting, have a broader range of activity, more potent, produce fewer side effects, more easily absorbed than, or have other useful pharmacological properties over, sitagliptin salts known in the prior art. Such advantages can also particularly occur during combination therapy with a further active ingredient e.g. a second anti-diabetic agent such as metformin, pioglitazone, or rosiglitazone or an anti-hypertensive agent such as valsartan or in combination with a statin.

The salts of the present invention may be solvent-free, preferentially free of water.

In yet another embodiment the sitagliptin salts of the present invention are in the form of hydrates of solvates, for example hemi-, mono-, di-, tri-, tetra-, penta-, or hexa-solvates or - hydrates, respectively. The solvents used for the crystallisation process, in particular alcohols, preferably methanol or ethanol, ketones, esters, preferably ethyl acetate, can be embedded in the crystal structure. Preferably the solvent is pharmaceutically acceptable.

According to a further aspect of the invention there is thus provided a pharmaceutical composition including a sitagliptin salt of the present invention, in admixture with a pharmaceutically acceptable excipient or carrier. Solid dosage forms for oral administration include capsules, tablets, pills, powders and granules. In such solid dosage forms, the active compound is typically mixed with at least one inert, pharmaceutically acceptable excipient or carrier, fillers or extenders, binders, humectants, disintegrating agents, and lubricants. Advantageously, the compounds of the invention may be orally active, have rapid onset of activity and low toxicity. A compound of the invention is preferably in the form of a tablet, preferably one obtainable by direct compression.

Figure 1 shows the XRPD pattern of sitagliptin HCl Form I.
Figure 2 shows the XRPD pattern of sitagliptin HCl Form II.
Figure 3 shows the XRPD pattern of sitagliptin fumarate Form I.
Figure 4 shows the XRPD pattern of sitagliptin fumarate Form II.
Figure 5 shows the XRPD pattern of sitagliptin malate.
Figure 6 shows the XRPD pattern of sitagliptin sulfate Form I
Figure 7 shows the XRPD-pattern of sitagliptin sulfate Form II.
Figure 8 shows the XRPD pattern of sitagliptin phosphate.
Figure 9 shows the XRPD pattern of sitagliptin succinate Form I.
Figure 10 shows the XRPD pattern of sitagliptin succinate Form II.
Figure 11 shows the XRPD pattern of sitagliptin succinate Form III.
Figure 12 shows the XRPD pattern of sitagliptin lactate.
Figure 13 shows the XRPD pattern of sitagliptin glycolate.
Figure 14 shows the XRPD pattern of sitagliptin maleate Form I.
Figure 15 shows the XRPD pattern of sitagliptin maleate Form II.
Figure 16 shows the XRPD pattern of sitagliptin citrate.
Figure 17 shows the XRPD pattern of sitagliptin mesylate Form I.
Figure 18 shows the XRPD pattern of sitagliptin mesylate Form II.

The invention will be further described by the following examples.

**EXAMPLES**

**Characterisation of samples**

The samples were characterized by X-ray powder diffraction (XRPD) and differential scanning calometry (DSC).

For XRD characterisation, the sample were, if necessary, grinded in a mortar from agate and properly prepared (flatted surface in z0) on a specimen holder made from PMMA. The samples were then analyzed on a Bruker-AXS D8 Advance powder X-ray diffractometer (Bruker-AXS, Karlsruhe, Germany). The specimen holder was rotated in the z0 plane at 20 rpm during measurement. The measurement conditions were as follows: Radiation: Cu K$\alpha$, Source 40 kV / 40 mA, divergence slit 0.6 mm, detector: Våntec-1 anti-scattering slit 5.59 mm, detector slit 10.28 mm, start angle 2°, end angle 55°, Step 0.016° 2θ. Raw data were evaluated using the program EVA (Bruker-AXS, Karlsruhe, Germany).

In the tables of the following examples the 2 theta angles are given with two ore more decimal places. However, the actual accuracy of the measurement is lower and, therefore, all 2 theta angles should be mathematically rounded to one decimal place. For example, a 2 theta angle of 6.562° is to be rounded to 6.6°. Moreover, in all cases an error of $\pm$ 0.2° is to be assumed. The same is true for the d value, which is given in Angström and which should be rounded to one decimal place and having an error of $\pm$ 0.2 Ångström.

A Mettler Toledo 822e DSC instrument was used to record the DSC curves. A sample amount of 1.5 to 2 mg was placed in the calorimeter cell and heated from 30 °C to 300 °C with a rate of 10 °C/min and a nitrogen flow of 50 ml/min. The results of the DSC experiments are summarized in tables 25-27.

**Example 1: Sitagliptin Hydrochloride Form I**

In a 250 ml round bottom two neck flask sitagliptin free base (8.0 g, 19.65 mmol) was dissolved in 80 ml isopropanol. The reaction was stirred on a magnetic stirrer to give a clear solution. Aqueous hydrochloric acid 2.4 ml (30%) was added drop wise over a period of 10 minutes and was allowed to stir at room temperature. In the next 15 minutes a thick white precipitate was observed. An additional 40 ml of isopropanol was added to facilitate the stirring for a further 20 minutes and was filtered and dried at 60 °C for 3 h at 5 mbar vacuum to obtain 8.0 g (92.4 % yield) of white powder of sitagliptin hydrochloride. The residual solvent content was 2.02 % isopropanol as determined by headspace GC.
DSC = 109.3 $\pm$ 2 °C and 171.0 $\pm$ 2 °C

Recrystallization

For recrystallization, a portion of the sitagliptin hydrochloride (7.5 g, white powder) was dissolved in 50 ml of ethanol by warming on water bath at 60 °C for a period of 15-20 minutes. The clear solution was kept at room temperature for overnight for crystallization. A thick cluster of white precipitate was observed in the flask and was filtered over a Büchner funnel and dried at 60 °C for 4h at 5 mbar vacuum on a rotary evaporator to obtain 6.27 g of Sitagliptin

hydrochloride.
DSC: 95.9 ± 2 °C; 173.3 °C ± 2 °C. HPLC purity = 99.99 %.
Specific optical rotation = -24.44°

The mother liquor of above reaction was kept overnight to yield more of the crystalline salt. It was filtered and dried similarly and yielded another 0.574 g.

Table 1: XRPD data of sitagliptin hydrochloride form I

| Angle | d value | Intensity |
|---|---|---|
| 2-Theta° | Angström | % |
| 6.562 | 13.45816 | 89.0 |
| 7.542 | 11.71200 | 94.4 |
| 7.820 | 11.29617 | 65.0 |
| 7.968 | 11.08642 | 46.9 |
| 9.521 | 9.28189 | 11.0 |
| 10.339 | 8.54899 | 15.7 |
| 12.305 | 7.18724 | 24.3 |
| 12.639 | 6.99787 | 12.3 |
| 12.758 | 6.93313 | 12.7 |
| 13.218 | 6.69309 | 28.3 |
| 13.698 | 6.45950 | 61.1 |
| 15.953 | 5.55095 | 30.8 |
| 16.393 | 5.40312 | 36.6 |
| 16.624 | 5.32850 | 38.4 |
| 17.779 | 4.98491 | 19.7 |
| 18.041 | 4.91293 | 51.9 |
| 18.653 | 4.75327 | 71.0 |
| 19.288 | 4.59803 | 96.6 |
| 19.524 | 4.54299 | 50.8 |
| 19.857 | 4.46766 | 62.2 |
| 20.063 | 4.42211 | 30.7 |
| 20.250 | 4.38177 | 30.2 |
| 20.457 | 4.33782 | 18.8 |
| 20.749 | 4.27748 | 24.0 |
| 22.616 | 3.92852 | 37.0 |
| 23.414 | 3.79627 | 21.1 |
| 23.817 | 3.73300 | 28.8 |
| 24.162 | 3.68042 | 19.5 |
| 24.419 | 3.64226 | 24.7 |
| 24.787 | 3.58903 | 69.9 |
| 25.403 | 3.50339 | 100.0 |
| 25.579 | 3.47975 | 82.2 |
| 25.911 | 3.43582 | 20.4 |

(continued)

| Angle | d value | Intensity |
|---|---|---|
| 2-Theta° | Angström | % |
| 26.615 | 3.34653 | 22.2 |
| 27.052 | 3.29353 | 65.6 |
| 27.675 | 3.22071 | 9.7 |
| 28.409 | 3.13917 | 27.3 |
| 28.977 | 3.07889 | 28.6 |
| 29.498 | 3.02567 | 34.5 |
| 29.833 | 2.99245 | 13.1 |
| 30.616 | 2.91771 | 22.0 |
| 31.102 | 2.87318 | 9.6 |
| 31.469 | 2.84055 | 17.2 |
| 31.635 | 2.82603 | 15.9 |
| 32.207 | 2.77712 | 13.0 |
| 32.537 | 2:74971 | 15.2 |
| 32.903 | 2.71993 | 14.3 |
| 33.356 | 2.68406 | 5.7 |
| 34.156 | 2.62300 | 8.7 |
| 34.849 | 2.57236 | 8.9 |
| 36.462 | 2.46220 | 6.5 |
| 37.631 | 2.38838 | 8.9 |

**Example 2: Sitagliptin Hydrochloride Form II**

Sitagliptin hydrochloride (2.0 g, form I from example 1) was dissolved in 125 ml of ethyl acetate in a two neck flask and warmed in an oil bath which was preheated at 125 °C for about 30 minutes. The solution turned clear and the flask was removed from the oil bath and allowed to cool to room temperature. A white precipitate crystallized after 10 minutes. 10 ml ethyl acetate was added to aid filtration & compound was filtered and dried at 70 °C for 6 h. Yield = 1.66 g. DSC = 202.3 °C ± 2 °C

In case product obtained is mixture of two forms of Sitagliptin hydrochloride, it should be recrystallised from ethyl acetate.

Table 2: XRPD data of sitagliptin hydrochloride form II

| Angle | d value | Intensity % |
|---|---|---|
| 2-Theta° | Angström | % |
| 6.258 | 14.11189 | 12.1 |
| 8.923 | 9.90222 | 12.7 |
| 12.542 | 7.05174 | 15.9 |
| 13.553 | 6.52816 | 100.0 |
| 14.607 | 6.05932 | 7.5 |
| 15.378 | 5.75731 | 9.4 |
| 16.739 | 5.29222 | 8.1 |

(continued)

| Angle | d value | Intensity % |
|---|---|---|
| 2-Theta° | Angström | % |
| 16.942 | 5.22913 | 17.9 |
| 17.740 | 4.99577 | 54.9 |
| 17.770 | 4.98742 | 53.5 |
| 18.252 | 4.85658 | 23.1 |
| 18.789 | 4.71902 | 42.8 |
| 19.870 | 4.46472 | 7.7 |
| 20.777 | 4.27177 | 10.3 |
| 21.376 | 4.15352 | 8.7 |
| 21.995 | 4.03784 | 15.8 |
| 22.483 | 3.95144 | 18.2 |
| 22.655 | 3.92172 | 13.0 |
| 23.579 | 3.77015 | 29.0 |
| 24.409 | 3.64383 | 12.7 |
| 24.776 | 3.59058 | 46.4 |
| 24.893 | 3.57396 | 67.2 |
| 25.189 | 3.53274 | 41.0 |
| 25.472 | 3.49402 | 18.8 |
| 25.945 | 3.43141 | 16.7 |
| 26.031 | 3.42035 | 17.6 |
| 26.941 | 3.30681 | 39.8 |
| 27.685 | 3.21955 | 12.5 |
| 27.810 | 3.20539 | 13.0 |
| 28.540 | 3.12510 | 11.5 |
| 29.024 | 3.07398 | 14.8 |
| 30.276 | 2.94967 | 9.3 |
| 30.729 | 2.90724 | 5.9 |
| 31.202 | 2.86424 | 10.1 |
| 31.597 | 2.82936 | 21.5 |
| 32.087 | 2.78720 | 11.6 |
| 34.015 | 2.63353 | 6.0 |
| 35.284 | 2.54164 | 5.5 |
| 37.607 | 2.38985 | 5.1 |
| 38.331 | 2.34637 | 5.0 |
| 38.826 | 2.31756 | 6.9 |
| 39.859 | 2.25985 | 5.2 |

**Example 3: Procedure for preparing Sitagliptin Fumarate Form I**

Sitagliptin free base (8.0 g, 19.65 mmol) was dissolved in 80 ml of ethyl acetate in a two neck flask. The reaction mixture was warmed at 50 °C for about 7 minutes on a water bath to obtain clear solution and stirred using a teflon blade/mechanical stirrer at 200 rpm.

In another flask, fumaric acid (2.286 g, 19.65 mmol) was dissolved in a mixture 80.0 ml of ethyl acetate and 60.0 ml of isopropanol and warmed over the water bath at 50 °C for 5 min to obtain a clear solution. Fumaric acid solution was transferred in a dropping funnel and added dropwise to the Sitagliptin free base solution over a period of 6 min with a stirring speed of 305 rpm. The temperature of the reaction was increased by 1.2 °C (25.5 to 26.7 °C). The clear solution turned turbid during addition and during the following five minutes a thick precipitate separated out. This was stirred for a period of 30 minutes and filtered and dried at 70 °C/ 3 h / 5 mbar vacuum to obtain 9.42 g (91%) of Sitagliptin fumarate. DSC = 177.0 °C ± 2 °C, total purity = 99.98 %. Specific optical rotation = -20.77

Table 3: XRPD data of Sitagliptin fumarate form I

| Angle | d value | Intensity |
|---|---|---|
| 2-Theta ° | Angstrom | % |
| 2.777 | 31.78772 | 0.4 |
| 3.255 | 27.12498 | 5.1 |
| 6.954 | 12.70151 | 2.5 |
| 7.962 | 11.09588 | 4.2 |
| 10.558 | 8.37241 | 11.3 |
| 11.459 | 7.71569 | 10.7 |
| 12.093 | 7.31258 | 3.1 |
| 13.270 | 6.66677 | 32.3 |
| 13.884 | 6.37337 | 28.6 |
| 15.645 | 5.65970 | 12.8 |
| 16.014 | 5.52987 | 18.5 |
| 16.917 | 5.23670 | 44.1 |
| 17.318 | 5.11643 | 100.0 |
| 17.860 | 4.96242 | 23.6 |
| 18.307 | 4.84223 | 16.7 |
| 19.907 | 4.45641 | 0.4 |
| 21.043 | 4.21831 | 23.5 |
| 22.467 | 3.95415 | 20.6 |
| 23.144 | 3.83994 | 11.6 |
| 24.253 | 3.66687 | 65.6 |
| 25.033 | 3.55432 | 24.9 |
| 26.502 | 3.36051 | 42.9 |
| 27.635 | 3.22527 | 16.8 |
| 28.194 | 3.16258 | 14.3 |
| 29.337 | 3.04192 | 14.4 |
| 30.904 | 2.89121 | 9.6 |
| 31.932 | 2.80038 | 8.6 |
| 32.329 | 2.76691 | 10.7 |

(continued)

| Angle | d value | Intensity |
|---|---|---|
| 2-Theta ° | Angstrom | % |
| 32.777 | 2.73009 | 6.6 |
| 34.209 | 2.61905 | 7.2 |
| 35.009 | 2.56099 | 4.0 |
| 35.524 | 2.52508 | 3.3 |
| 36.840 | 2.43783 | 8.1 |
| 37.247 | 2.41211 | 8.5 |
| 37.766 | 2.38014 | 5.3 |

**Example 4: Procedure for preparing Sitagliptin Fumarate Form II**

Sitagliptin fumarate (1.6 g, form I derived according example 3) was dissolved in 35.0 ml of ethanol and was stirred over a magnetic stirrer. Within 10 min the clear solution obtained turned turbid and started crystallizing out. The mixture was stirred for 3 h at room temperature; 20 ml ethanol was added and filtered. The filter cake was dried at 70 °C for 4 hours. Yield = 1.547 g. DSC = 188.36 ± 2 °C.

Table 4: XRPD data of sitagliptin fumarate form II

| Angle | d value | Intensity |
|---|---|---|
| 2-Theta° | Angström | % |
| 6.217 | 14.20434 | 38.1 |
| 7.109 | 12.42491 | 36.4 |
| 12.541 | 7.05265 | 34.7 |
| 12.953 | 6.82892 | 23.7 |
| 14.355 | 6.16502 | 47.5 |
| 14.840 | 5.96468 | 40.7 |
| 15.878 | 5.57692 | 81.4 |
| 16.914 | 5.23779 | 40.7 |
| 17.812 | 4.97558 | 30.5 |
| 18.005 | 4.92268 | 27.1 |
| 19.103 | 4.64230 | 100.0 |
| 20.007 | 4.43442 | 46.6 |
| 20.189 | 4.39482 | 43.2 |
| 21.402 | 4.14838 | 42.4 |
| 21.679 | 4.09614 | 51.7 |
| 21.877 | 4.05940 | 55.9 |
| 24.000 | 3.70492 | 57.6 |
| 25.264 | 3.52236 | 91.5 |
| 26.037 | 3.41949 | 62.7 |
| 26.472 | 3.36426 | 43.2 |
| 27.398 | 3.25271 | 26.3 |

(continued)

| Angle | d value | Intensity |
|---|---|---|
| 2-Theta° | Angström | % |
| 28.276 | 3.15362 | 17.8 |
| 28.699 | 3.10815 | 22.0 |
| 29.077 | 3.06857 | 26.3 |
| 29.560 | 3.01952 | 29.7 |
| 29.848 | 2.99100 | 32.2 |
| 31.732 | 2.81764 | 28.8 |
| 33.154 | 2.69996 | 16.9 |

**Example 5: Sitagliptin Malate**

In a two neck flask Sitagliptin free base (7.0 g, 17.199 mmol) was dissolved in 70 ml of ethyl acetate and warmed at 60 °C on a water bath to obtain a clear solution while stirring on a magnetic stirrer. In another flask L-malic acid (2.306 g, 17.199 mmol) and 14.0 ml of ethanol were mixed and warmed on the water bath at 60 °C for 2 min to obtain a clear solution. The clear solution of malic acid was transferred in a dropping funnel and added dropwise to the solution of Sitagliptin free base over a period of 5 min. The clear solution was seeded with a crystalline sitagliptin malate sample derived from a previous experiment and stored for the crystallization. After two days the crystallized salt was scraped, filtered and dried at 70 °C/ 3 h / 5 mbar vacuum to obtain 8.7 g of Sitagliptin malate.
DSC shows two peaks at 136.6 ± 2 °C (minor peak) and 153.5 ± 2 °C (major peak).

Purification

8.50 g of Sitagliptin malate (solid white powder) was dissolved in 150 ml of ethyl acetate in a two neck round bottom flask. This flask was placed for stirring over a mechanical stirrer and was placed in an oil bath which was preheated at 80 °C and stirred for one hour with the rotation speed of 313 rpm. The reaction mixture was allowed to cool to room temperature. The solid obtained was filtered over a Büchner funnel and dried under vacuum of 5 mbar at 60 °C / 3 h /, 70 °C /1 h and 80 °C / 1h to obtain 8.25 g of Sitagliptin malate.
DSC = 154.4 ± 2 °C indicating a pure form of Sitagliptin malate.
HPLC: Area % due to malic acid = 2.44 %; Area % due to Sitagliptin = 97.55 %
Total purity = 99.99 %

Table 5: XRPD-data of sitagliptin malate, grinded

| Angle | d value | Intensity |
|---|---|---|
| 2-Theta° | Angström | % |
| 8.38 | 10.54 | 9.7 |
| 10.07 | 8.78 | 5.2 |
| 12.44 | 7.11 | 22.4 |
| 12.96 | 6.83 | 10.8 |
| 14.13 | 6.26 | 16.7 |
| 15.08 | 5.87 | 17.8 |
| 15.86 | 5.58 | 100.0 |
| 16.84 | 5.26 | 43.8 |
| 17.01 | 5.21 | 41.7 |
| 17.95 | 4.94 | 61.4 |
| 18.30 | 4.84 | 19.1 |

(continued)

| Angle | d value | Intensity |
|---|---|---|
| 2-Theta° | Angström | % |
| 19.38 | 4.58 | 37.1 |
| 20.27 | 4.38 | 22.5 |
| 20.54 | 4.32 | 26.2 |
| 20.96 | 4.24 | 22.2 |
| 21.21 | 4.19 | 8.7 |
| 22.50 | 3.95 | 27.6 |
| 23.23 | 3.83 | 61.0 |
| 24.06 | 3.70 | 13.9 |
| 24.25 | 3.67 | 8.3 |
| 24.93 | 3.57 | 25.8 |
| 25.29 | 3.52 | 49.5 |
| 26.08 | 3.41 | 35.1 |
| 26.36 | 3.38 | 14.4 |
| 27.19 | 3.28 | 11.7 |
| 28.52 | 3.13 | 21.5 |
| 28.91 | 3.09 | 25.1 |
| 30.17 | 2.96 | 11.8 |
| 30.41 | 2.94 | 8.6 |
| 31.55 | 2.83 | 6.0 |
| 33.36 | 2.68 | 5.5 |
| 33.84 | 2.65 | 11.2 |

**Example 6: Sitagliptin Sulfate Form I**

In a two neck flask Sitagliptin free base (8.0 g, 19.65 mmol) was dissolved in 50 ml of ethanol. The reaction mass was warmed at 50 °C on a water bath for 5 min to obtain a clear solution and was placed over a magnetic stirrer. In another flask sulfuric acid (98%, 0.9639 g, 9.828 mmol) and 30.0 ml of ethanol were mixed to make a clear solution. The clear solution of $H_2SO_4$ was transferred in a dropping funnel and added dropwise over to a solution of Sitagliptin free base over a period of 6 min. The reaction mixture was stirred for 10 minutes and a white precipitate started emerging out and turned thick. The mixture was allowed to stir for another 30 minutes, filtered and dried at 60 °C / 1h / 5 mbar , 70 °C / 2 h / 5 mbar and 80 °C / 3h at 5 mbar vacuum, to obtain 8.50 g of Sitagliptin sulfate (94.8 % yield).

HPLC = 100 % ; DSC = 148.2 °C ± 2 °C (major peak) and 197.4 °C ± 2 °C (minor). Residual solvent analysis showed the presence of 3.26 % ethanol indicating this sitagliptin sulfate form I to be an ethanol solvate.

Table 6: XRPD data of sitagliptin sulfate form I

| Angle | d value | Intensity % |
|---|---|---|
| 2-Theta° | Angström | % |
| 4.51 | 19.58 | 9.1 |
| 5.26 | 16.80 | 20.9 |
| 5.92 | 14.92 | 9.7 |
| 7.77 | 11.37 | 5.6 |

(continued)

| Angle | d value | Intensity % |
|---|---|---|
| 2-Theta° | Angström | % |
| 11.79 | 7.50 | 10.5 |
| 12.45 | 7.10 | 15.7 |
| 13.11 | 6.75 | 14.8 |
| 13.50 | 6.55 | 14.0 |
| 14.68 | 6.03 | 12.8 |
| 15.78 | 5.61 | 48.8 |
| 16.73 | 5.30 | 53.5 |
| 17.35 | 5.11 | 35.1 |
| 18.02 | 4.92 | 7.0 |
| 18.84 | 4.71 | 51.7 |
| 19.35 | 4.58 | 25.1 |
| 20.20 | 4.39 | 32.9 |
| 21.22 | 4.18 | 73.8 |
| 21.66 | 4.10 | 100.0 |
| 22.96 | 3.87 | 27.4 |
| 24.33 | 3.66 | 13.4 |
| 25.11 | 3.54 | 18.8 |
| 25.76 | 3.46 | 14.8 |
| 26.34 | 3.38 | 11.0 |
| 27.05 | 3.29 | 26.3 |
| 27.63 | 3.23 | 9.2 |
| 29.56 | 3.02 | 5.0 |
| 30.26 | 2.95 | 10.1 |
| 30.98 | 2.88 | 11.6 |
| 31.56 | 2.83 | 5.1 |
| 34.89 | 2.57 | 6.3 |

**Example 7: Sitagliptin Sulfate Form II**

Sitagliptin sulfate (0.530 g, derived from example 6) was dissolved in 0.5 ml of water and the solution was kept in a freezer for 2 h and then kept at ambient temperature for 2 days for crystallization. The compound was dried 60 °C for 2 h and 40 °C for overnight.

Yield = 0.470 g. DSC = 101.1 °C ± 2 °C; 175.9 °C ± 2 °C (major), 195.9 °C ± 2 °C (minor). Residual solvent analysis by HPLC showed that this product was not a solvate.

Table 7: XRPD data of Sitagliptin sulfate Form II

| Angle | d value | Intensity % |
|---|---|---|
| 2-Theta ° | Angstrom | % |
| 5.650 | 15.62911 | 97.9 |
| 7.315 | 12.07553 | 54.1 |

(continued)

| Angle | d value | Intensity % |
|---|---|---|
| 2-Theta ° | Angstrom | % |
| 9.602 | 9.20380 | 43.3 |
| 12.455 | 7.10130 | 42.1 |
| 13.455 | 6.57567 | 59.5 |
| 14.154 | 6.25214 | 75.5 |
| 14.929 | 5.92941 | 55.2 |
| 15.070 | 5.87406 | 54.4 |
| 15.545 | 5.69583 | 60.7 |
| 15.923 | 5.56148 | 62.2 |
| 16.505 | 5.36661 | 60.0 |
| 17.383 | 5.09744 | 81.5 |
| 18.360 | 4.82831 | 82.9 |
| 19.167 | 4.62676 | 82.5 |
| 19.593 | 4.52724 | 92.6 |
| 20.251 | 4.38162 | 81.5 |
| 21.047 | 4.21761 | 86.4 |
| 22.189 | 4.00308 | 71.1 |
| 22.867 | 3.88592 | 100.0 |
| 24.319 | 3.65712 | 80.6 |
| 24.735 | 3.59654 | 92.8 |
| 28.309 | 3.15004 | 53.8 |
| 29.679 | 3.00764 | 57.9 |
| 30.398 | 2.93815 | 52.5 |
| 39.913 | 2.25694 | 31.0 |

**Example 8: Sitagliptin Phosphate**

In a two neck flask Sitagliptin free base (8.0 g, 19.65 mmol) was dissolved in 20 ml of ethanol and warmed at 50 °C on a water bath for 5 min to obtain a clear solution. The clear solution was stirred at room temperature using a magnetic stirrer.

In another flask phosphoric acid (88 %, 1.920 g; 19.591 mmol) was mixed with 5.0 ml of ethanol to make a clear solution. The clear solution of $H_3PO_4$ was transferred to a dropping funnel and added dropwise to a solution of Sitagliptin free base over a period of 10 min. During the addition of the $H_3PO_4$, a thick precipitate was observed hence an additional 30 ml of ethanol was added to the reaction mass to ensure proper stirring. The temperature of the reaction mixture increased by 3.0 °C (26.0 to 29.0 °C) during the addition of $H_3PO_4$ solution. The reaction mixture was stirred for 30 minutes and filtered. The obtained crystals were dried at 60 °C for 1h and at 70 °C for 3 h at 5 mbar vacuum, to obtain 7.58 g of Sitagliptin phosphate (94.8 %).
DSC: 213.7 °C ± 2 °C, HPLC: 99.67 %

Table 8: XRPD data of sitagliptin phosphate

| Angle | d value | Intensity |
|---|---|---|
| 2-Theta° | Angström | % |
| 3.643 | 24.23111 | 3.6 |
| 3.966 | 22.25976 | 5.2 |
| 4.656 | 18.96408 | 100.0 |
| 11.844 | 7.46619 | 4.8 |
| 12.339 | 7.16758 | 29.2 |
| 12.676 | 6.97766 | 7.5 |
| 13.534 | 6.53703 | 16.3 |
| 13.934 | 6.35049 | 32.2 |
| 15.137 | 5.84832 | 17.8 |
| 17.811 | 4.97589 | 7.0 |
| 18.313 | 4.84073 | 43.3 |
| 18.624 | 4.76060 | 13.7 |
| 19.468 | 4.55604 | 7.1 |
| 20.053 | 4.42442 | 15.6 |
| 20.534 | 4.32185 | 14.5 |
| 21.572 | 4.11612 | 6.1 |
| 22.042 | 4.02938 | 8.4 |
| 22.665 | 3.92014 | 4.1 |
| 23.724 | 3.74738 | 31.7 |
| 24.347 | 3.65294 | 26.4 |
| 25.506 | 3.48946 | 22.8 |
| 26.789 | 3.32522 | 27.7 |
| 27.122 | 3.28509 | 12.9 |
| 27.568 | 3.23300 | 5.9 |
| 28.113 | 3.17150 | 4.0 |
| 28.654 | 3.11293 | 3.4 |
| 30.163 | 2.96046 | 9.1 |
| 30.646 | 2.91490 | 4.2 |
| 32.729 | 2.73405 | 4.9 |
| 33.026 | 2.71009 | 7.5 |
| 33.293 | 2.68896 | 5.6 |

**Example 9: Sitagliptin Succinate Form I and Form II**

In a two neck flask sitagliptin free base (10.0 g, 24.570 mmol) was dissolved in 30 ml of ethyl acetate and warmed to 70 °C over a water bath to obtain clear solution and stirred using a magnetic stirrer. In another flask succinic acid (2.9 g, 24.557 mmol) was dissolved in 16 ml of ethanol and warmed over the water bath at 50 °C for 5 min to obtain a clear solution. This solution was transferred in a dropping funnel and added dropwise over a period of 5 min to the sitagliptin free base solution. To the clear solution 22.0 ml of n-hexane were added and the mixture was stored for crystallization.

The first crop was 1.95 g; having a DSC peak at 126.1 °C ± 2 °C which is characteristic of sitagliptin succinate Form I.
The second crop yielded 2.46 g. DSC showed peaks at 122.9 ± 2 °C (major) and 135.1 ± 2 °C indicating Form I contaminated with trace of Form II.
The third crop yielded 4.90 g with DSC peaks at 124.2 ± 2 °C and 136.3 ± 2 °C in ratio of 75 :25 of Form I and Form II respectively.
The fourth crop was 0.2 g with a DSC peak at 137.0 °C ± 2 °C (sitagliptin succinate Form II).

**Table 9 XRPD data of Sitagliptin succinate Form I**

| Angle | d value | Intensity |
|---|---|---|
| 2-Theta° | Angstrom | % |
| 10.5 | 8.44 | 7.7 |
| 11.5 | 7.67 | 9.4 |
| 13.1 | 6.76 | 19.2 |
| 13.5 | 6.54 | 26.4 |
| 14.1 | 6.27 | 15.7 |
| 15.7 | 5.63 | 21.7 |
| 17.1 | 5.18 | 36.8 |
| 17.4 | 5.08 | 100 |
| 17.6 | 5.04 | 65.4 |
| 18.1 | 4.90 | 5.4 |
| 18.6 | 4.78 | 3.3 |
| 20.2 | 4.40 | 23.7 |
| 20.6 | 4.30 | 6.6 |
| 21.1 | 41.98 | 8 |
| 21.8 | 4.07 | 9.7 |
| 23.0 | 3.86 | 9.1 |
| 23.8 | 3.73 | 18.2 |
| 24.4 | 36.52 | 41 |
| 25.2 | 3.54 | 20.4 |
| 25.8 | 3.45 | 34.3 |
| 26.4 | 3.38 | 21.6 |
| 27.1 | 3.29 | 8.7 |
| 28.0 | 3.18 | 10.8 |
| 28.7 | 3.11 | 4.7 |
| 29.2 | 3.06 | 5.4 |
| 29.6 | 3.01 | 3 |
| 30.5 | 2.93 | 5.3 |
| 31.2 | 2.87 | 3.5 |
| 31.7 | 2.82 | 6.6 |
| 33.2 | 2.70 | 2.6 |
| 34.5 | 2.60 | 5.6 |

(continued)

| Angle | d value | Intensity |
|---|---|---|
| 2-Theta° | Angstrom | % |
| 35.6 | 2.52 | 2.2 |
| 36.5 | 2.46 | 2.5 |
| 2-Theta ° | Angstrom | % |
| 37.7 | 2.38 | 5.3 |
| 38.9 | 2.32 | 3.2 |
| 39.3 | 2.29 | 2.9 |

**Table 10 XRPD data of Sitagliptin succinate Form II**

| Angle | d value | Intensity % |
|---|---|---|
| 2-Theta ° | Angstrom | % |
| 6.173 | 14.30681 | 52.2 |
| 7.063 | 12.50599 | 50.7 |
| 9.394 | 9.40687 | 32.4 |
| 10.832 | 8.16091 | 31.2 |
| 12.449 | 7.10466 | 37.0 |
| 12.662 | 6.98551 | 42.4 |
| 12.872 | 6.87219 | 38.7 |
| 13.706 | 6.45584 | 38.6 |
| 14.245 | 6.21272 | 48.7 |
| 14.506 | 6.10153 | 44.7 |
| 14.860 | 5.95664 | 44.9 |
| 15.952 | 5.55127 | 77.9 |
| 16.318 | 5.42777 | 50.9 |
| 17.113 | 5.17725 | 60.9 |
| 17.721 | 5.00093 | 55.4 |
| 18.164 | 4.87995 | 51.4 |
| 19.007 | 4.66553 | 100.0 |
| 20.316 | 4.36775 | 62.1 |
| 20.590 | 4.31021 | 59.1 |
| 21.337 | 4.16095 | 64.4 |
| 21.899 | 4.05545 | 78.1 |
| 23.271 | 3.81931 | 57.5 |
| 23.661 | 3.75723 | 65.3 |
| 24.076 | 3.69348 | 80.3 |
| 24.571 | 3.62010 | 65.4 |
| 25.055 | 3.55133 | 76.1 |

(continued)

| Angle | d value | Intensity % |
|---|---|---|
| 2-Theta ° | Angstrom | % |
| 25.904 | 3.43682 | 59.4 |
| 26.172 | 3.40212 | 70.9 |
| 26.504 | 3.36026 | 62.0 |
| 26.955 | 3.30517 | 51.7 |
| 27.580 | 3.23163 | 48.7 |
| 28.239 | 3.15768 | 46.4 |
| 28.575 | 3.12125 | 45.2 |
| 29.316 | 3.04407 | 47.7 |
| 29.812 | 2.99452 | 52.6 |
| 30.112 | 2.96536 | 47.0 |
| 30.701 | 2.90980 | 39.0 |
| 31.490 | 2.83874 | 46.0 |
| 32.292 | 2.76997 | 38.5 |
| 33.126 | 2.70213 | 39.4 |
| 36.517 | 2.45861 | 33.1 |

**Example 10: Sitagliptin Succinate Form III**

In a two neck flask sitagliptin free base (4.0 g, 9.828 mmol) was dissolved in 12.0 ml of ethyl acetate and warmed to 60 °C over a water bath to obtain clear solution and stirred using a magnetic stirrer. In another flask succinic acid (1.16 g, 10.0 mmol) was dissolved in 6 ml of ethanol and warmed over the water bath at 50 °C for 5 min to obtain a clear solution. This solution was transferred in a dropping funnel and added dropwise over a period of 5 min to the sitagliptin free base solution. To the clear solution 8.8 ml of n-hexane were added and the mixture was seeded with a crystalline sitagliptin succinate salt (derived from example 9) and stored for crystallization. This precipitate was filtered and dried at 60 °C for 3 h. This yielded 4.9 g of sitagliptin succinate.

**Purification**

The sitagliptin succinate salt of this example can, if desired, be further purified to remove traces of an impurity of another polymorphic form having a DSC peak at 133.3 °C ± 2 °C.

To 4.5 g of the isolated sitagliptin succinate salt of this example was added 50 ml of ethyl acetate and stirred at room temperature for 2 h and was filtered and dried at 60 °C for 3 h. The yield was 4.2 g sitagliptin succinate having a DSC peak at 167.8 °C ± 2 °C.

Table 11 XRPD data of Sitagliptin succinate Form III all

| Angle | d value | Intensity % |
|---|---|---|
| 2-Theta ° | Angstrom | % |
| 3.681 | 23.98247 | 10.6 |
| 4.140 | 21.32625 | 11.3 |
| 5.953 | 14.83359 | 14.8 |
| 8.375 | 10.54906 | 46.0 |
| 8.812 | 10.02709 | 8.0 |
| 9.431 | 9.37044 | 9.3 |

(continued)

| Angle | d value | Intensity % |
|---|---|---|
| 2-Theta ° | Angstrom | % |
| 11.942 | 7.40512 | 23.2 |
| 13.453 | 6.57621 | 18.6 |
| 15.216 | 5.81810 | 20.9 |
| 16.806 | 5.27119 | 31.8 |
| 17.340 | 5.10990 | 63.3 |
| 17.814 | 4.97509 | 17.0 |
| 18.863 | 4.70068 | 31.8 |
| 19.287 | 4.59838 | 22.2 |
| 20.484 | 4.33232 | 9.0 |
| 21.173 | 4.19271 | 100.0 |
| 21.529 | 4.12423 | 24.8 |
| 22.749 | 3.90581 | 45.7 |
| 23.002 | 3.86332 | 18.0 |
| 24.066 | 3.69490 | 13.8 |
| 24.704 | 3.60091 | 13.2 |
| 24.917 | 3.57065 | 13.8 |
| 25.342 | 3.51174 | 22.8 |
| 26.089 | 3.41276 | 10.0 |
| 27.176 | 3.27873 | 28.0 |
| 27.650 | 3.22361 | 7.7 |
| 28.406 | 3.13947 | 9.0 |
| 30.095 | 2.96704 | 8.7 |
| 30.896 | 2.89193 | 15.4 |
| 31.209 | 2.86366 | 7.7 |
| 32.233 | 2.77495 | 11.6 |
| 32.374 | 2.76318 | 11.9 |
| 33.272 | 2.69060 | 7.7 |
| 33.456 | 2.67625 | 8.7 |
| 34.026 | 2.63270 | 9.6 |
| 34.488 | 2.59848 | 12.5 |
| 34.902 | 2.56859 | 10.0 |
| 35.310 | 2.53984 | 8.7 |
| 35.617 | 2.51868 | 7.7 |
| 36.476 | 2.46127 | 7.4 |
| 36.902 | 2.43384 | 8.7 |
| 38.244 | 2.35151 | 7.1 |
| 39.256 | 2.29316 | 8.4 |

(continued)

| Angle | d value | Intensity % |
|---|---|---|
| 2-Theta ° | Angstrom | % |
| 39.670 | 2.27019 | 6.4 |
| 40.412 | 2.23018 | 6.4 |
| 40.891 | 2.20515 | 7.7 |
| 41.146 | 2.19211 | 7.1 |
| 42.112 | 2.14400 | 5.8 |
| 42.372 | 2.13146 | 6.1 |
| 44.212 | 2.04692 | 6.8 |
| 44.468 | 2.03571 | 6.1 |
| 45.546 | 1.99002 | 6.1 |
| 46.321 | 1.95852 | 5.8 |
| 46.424 | 1.95439 | 5.8 |
| 47.014 | 1.93126 | 5.5 |
| 47.862 | 1.89899 | 5.5 |
| 49.030 | 1.85646 | 5.1 |
| 49.308 | 1.84662 | 6.1 |
| 51.394 | 1.77648 | 4.5 |

**Example 11: Sitagliptin Lactate**

In a two neck flask sitagliptin free base (10.0 g, 24.57 mmol) was dissolved in 40 ml of ethyl acetate and warmed to 50 °C over a water bath to obtain clear solution and stirred using a magnetic stirrer. In another flask L-lactic acid (88%, 2.5 g, 24.20 mmol) was dissolved in 10.0 ml of ethyl acetate to obtain a clear solution. This solution was transferred into a dropping funnel and added dropwise over a period of 5-7 min. The clear solution began turning turbid in the first 10 min then became a thick precipitate with an increase of temperature of the reaction mixture by 2 °C. To filter this precipitate an additional 60.0 ml of ethyl acetate can be added and filtered. Drying was done at 60 °C for 1h and at 80 °C for 2h at 1 mbar vacuum.
Yield 7.75 g. DSC peak at 150.3 °C ± 2 °C. Specific optical rotation = -25.28°

Table 12 XRPD data of Sitagliptin lactate

| Angle | d value | Intensity % |
|---|---|---|
| 2-Theta ° | Angstrom | % |
| 5.985 | 14.75563 | 18.9 |
| 8.164 | 10.82121 | 39.2 |
| 9.877 | 8.94769 | 2.6 |
| 10.603 | 8.33674 | 87.1 |
| 11.981 | 7.38084 | 4.5 |
| 12.344 | 7.16493 | 2.4 |
| 14.880 | 5.94898 | 16.5 |
| 15.617 | 5.66955 | 2.7 |
| 16.350 | 5.41716 | 8.9 |

(continued)

| Angle | d value | Intensity % |
|---|---|---|
| 2-Theta ° | Angstrom | % |
| 17.123 | 5.17429 | 26.2 |
| 17.757 | 4.99104 | 100.0 |
| 19.499 | 4.54876 | 12.6 |
| 20.225 | 4.38714 | 40.5 |
| 20.810 | 4.26501 | 58.6 |
| 21.189 | 4.18961 | 58.3 |
| 22.374 | 3.97035 | 7.3 |
| 23.336 | 3.80877 | 18.5 |
| 24.149 | 3.68247 | 16.3 |
| 24.926 | 3.56943 | 47.3 |
| 25.515 | 3.48833 | 17.9 |
| 26.294 | 3.38673 | 16.3 |
| 26.761 | 3.32863 | 27.0 |
| 27.043 | 3.29456 | 23.8 |
| 27.703 | 3.21758 | 15.3 |
| 29.956 | 2.98051 | 16.1 |
| 30.216 | 2.95546 | 14.4 |
| 32.371 | 2.76342 | 9.3 |
| 32.929 | 2.71786 | 12.4 |
| 33.344 | 2.68501 | 11.4 |
| 34.317 | 2.61103 | 7.1 |
| 35.052 | 2.55795 | 5.2 |
| 36.524 | 2.45820 | 8.4 |
| 41.041 | 2.19747 | 2.8 |
| 44.071 | 2.05314 | 2.7 |
| 45.732 | 1.98237 | 2.6 |
| 47.758 | 1.90287 | 3.7 |
| 48.273 | 1.88379 | 3.9 |
| 15.040 | 5.88579 | 7.2 |
| 16.829 | 5.26387 | 17.0 |
| 24.671 | 3.60566 | 36.6 |
| 28.040 | 3.17966 | 14.6 |
| 39.362 | 2.28724 | 10.2 |
| 37.964 | 2.36817 | 5.6 |

**Example 12: Sitagliptin Glycolate**

In a two neck flask sitagliptin free base (7.0 g, 17.99 mmol) was dissolved in 70 ml of isopropanol and warmed

to 50 °C over a water bath to obtain clear solution and stirred using a magnetic stirrer. In another flask glycolic acid (1.307 g, 17.99 mmol) was dissolved in 21.0 ml of isopropanol and warmed over the water bath at 50 °C for 5 min to obtain a clear solution. This solution was transferred into a dropping funnel and added dropwise over a period of 5-7 min. The clear solution turned turbid in the first 10 min and a thick mass precipitated. To filter this precipitate an additional amount of 50.0 ml of isopropanol can be added. The mixture was filtered and dried at 60 °C for 1 h, at 50 °C for 6h and at room temperature overnight at 1 mbar vacuum. Yield =7.019 g. DSC peaks at 89.1 °C ± 2 °C and 101.6 °C ± 2 °C were observed. The residual solvent analysis showed the presence of 5.93 % isopropanol indicating that this sitagliptin glycolate was an isopropanol solvate.

Table 13 XRPD data of Sitagliptin glycolate

| **Angle** | **d value** | **Intensity** |
|---|---|---|
| **2-Theta °** | **Angstrom** | **%** |
| 6.0 | 14.65 | 12.2 |
| 6.5 | 13.69 | 37.9 |
| 7.3 | 12.06 | 100.0 |
| 11.8 | 7.49 | 16.3 |
| 14.9 | 5.93 | 20.1 |
| 15.5 | 5.73 | 10.9 |
| 16.0 | 5.55 | 6.4 |
| 17.0 | 5.21 | 16.5 |
| 18.1 | 4.90 | 17.2 |
| 18.8 | 4.71 | 7.1 |
| 19.5 | 4.55 | 21.4 |
| 20.1 | 4.41 | 22.8 |
| 20.4 | 4.35 | 27.5 |
| 20.7 | 4.29 | 11.3 |
| 21.1 | 4.21 | 10.7 |
| 22.2 | 4.00 | 14.2 |
| 23.9 | 3.73 | 8.1 |
| 24.5 | 3.63 | 6.4 |
| 24.9 | 3.58 | 10.9 |
| 25.1<br>26.1 | 3.55<br>3.41 | 10.3<br>22,2 |

**Example 13: Sitagliptin Maleate Form I**

In a two neck flask sitagliptin free base (7.0 g, 17.19 mmol) was dissolved in 16 ml of ethanol and warmed to 50 °C over a water bath to obtain clear solution and stirred using a magnetic stirrer. In another flask maleic acid (1.996 g, 17.19 mmol) was dissolved in 4.0 ml of ethanol and warmed over the water bath at 50 °C for 5 min to obtain a clear solution. This solution was transferred into a dropping funnel and added drop wise to the sitagliptin solution over a period of 5 min with a stirring speed of 305 rpm. The clear solution was kept overnight for crystallization. Optionally, the solution can be seeded with sitagliptin maleate salt crystals derived from a previous experiment. On the next day the solid had separated out and was filtered. 50 ml diisopropyl ether can be used to aid the filtration. The filter cake was dried at 50 °C for 1 h and at 60 °C for 1 h at 5 mbar vacuum to obtain 9.00 g of sitagliptin maleate.

Purification

160 ml of diisopropyl ether was added to 9 mg of Sitagliptin maleate and the suspension was stirred vigorously for 24 hrs at ambient temperature. 100 ml of diisopropyl ether was added and the solid was filtered and dried at 60 °C under vacuum for 10 hours.

DSC = 92.5°C ± 2°C.

Residual solvent analysis showed 5.76 % ethanol and 1.07 % diisopropyl ether.

Table 14: XRPD data of Sitagliptin maleate Form I

| Angle | d value | Intensity % |
|---|---|---|
| **2-Theta °** | **Angstrom** | **%** |
| 4.062 | 21.73683 | 11.0 |
| 5.621 | 15.71097 | 100.0 |
| 6.841 | 12.91039 | 27.6 |
| 8.148 | 10.84267 | 7.6 |
| 8.917 | 9.90866 | 8.5 |
| 9.597 | 9.20809 | 7.1 |
| 9.840 | 8.98160 | 7.5 |
| 11.293 | 7.82933 | 34.2 |
| 13.810 | 6.40735 | 18.7 |
| 14.493 | 6.10664 | 18.7 |
| 16.074 | 5.50945 | 9.1 |
| 16.494 | 5.37026 | 24.0 |
| 17.110 | 5.17809 | 14.0 |
| 17.853 | 4.96437 | 52.0 |
| 18.406 | 4.81628 | 20.4 |
| 18.800 | 4.71630 | 12.0 |
| 19.356 | 4.58220 | 32.7 |
| 20.847 | 4.25771 | 21.5 |
| 21.253 | 4.17725 | 10.9 |
| 21.887 | 4.05761 | 10.6 |
| 22.775 | 3.90142 | 40.3 |
| 23.258 | 3.82150 | 20.0 |
| 23.687 | 3.75312 | 14.5 |
| 24.903 | 3.57259 | 55.3 |
| 26.203 | 3.39822 | 14.5 |
| 26.501 | 3.36068 | 10.7 |
| 27.130 | 3.28416 | 9.3 |
| 27.814 | 3.20496 | 13.7 |
| 28.589 | 3.11986 | 10.0 |

(continued)

| Angle | d value | Intensity % |
|---|---|---|
| 2-Theta ° | Angstrom | % |
| 29.043 | 3.07208 | 8.9 |
| 29.544 | 3.02113 | 9.0 |
| 30.465 | 2.93188 | 10.5 |
| 30.955 | 2.88649 | 7.0 |
| 31.738 | 2.81705 | 7.2 |
| 32.476 | 2.75473 | 8.0 |
| 32.769 | 2.73080 | 7.1 |
| 33.428 | 2.67845 | 7.6 |
| 34.439 | 2.60211 | 8.9 |
| 36.100 | 2.48608 | 7.8 |
| 36.251 | 2.47603 | 8.2 |
| 37.134 | 2.41918 | 7.6 |
| 39.489 | 2.28019 | 6.3 |
| 39.934 | 2.25577 | 6.2 |

**Example 14: Preparation of Sitagliptin Maleate Form II**

In a two neck flask sitagliptin maleate (2.6 g, from example 13) was suspended in 50 ml of ethyl acetate and was stirred over a magnetic stirrer for 30 min and allowed to stand for 1 h. Sitagliptin maleate precipitated and was filtered. 10 ml of ethyl acetate was added to aid the filtration and the filter cake was dried at 60 °C for 3 h.

The yield was 2.54 g sitagliptin maleate having a DSC peak at 159.5 °C ± 2 °C.

Table 15: XRPD data of Sitagliptin maleate Form 11

| Angle | d value | Intensity |
|---|---|---|
| 2-Theta° | Angström | % |
| 3.681 | 23.98380 | 100.0 |
| 6.369 | 13.86546 | 14.0 |
| 7.366 | 11.99130 | 15.8 |
| 9.755 | 9.05949 | 42.5 |
| 11.065 | 7.98995 | 8.0 |
| 12.788 | 6.91715 | 9.0 |
| 13.311 | 6.64645 | 6.4 |
| 14.777 | 5.99013 | 5.8 |
| 16.118 | 5.49458 | 37.6 |
| 16.966 | 5.22174 | 8.6 |
| 17.173 | 5.15942 | 7.4 |
| 17.960 | 4.93494 | 26.5 |
| 18.342 | 4.83298 | 34.4 |
| 18.490 | 4.79463 | 17.8 |

(continued)

| Angle | d value | Intensity |
|---|---|---|
| 2-Theta° | Angström | % |
| 19.241 | 4.60919 | 24.2 |
| 19.447 | 4.56088 | 35.4 |
| 19.594 | 4.52688 | 18.4 |
| 20.184 | 4.39603 | 6.2 |
| 20.639 | 4.30013 | 21.2 |
| 21.152 | 4.19686 | 9.9 |
| 21.476 | 4.13439 | 10.0 |
| 22.280 | 3.98694 | 10.8 |
| 22.577 | 3.93510 | 20.2 |
| 23.182 | 3.83372 | 55.5 |
| 23.939 | 3.71425 | 44.1 |
| 24.362 | 3.65075 | 19.6 |
| 25.104 | 3.54448 | 8.9 |
| 25.692 | 3.46460 | 27.8 |
| 25.908 | 3.43630 | 39.1 |
| 26.777 | 3.32666 | 13.4 |
| 28.047 | 3.17889 | 11.8 |
| 28.224 | 3.15930 | 11.2 |
| 29.148 | 3.06123 | 5.8 |
| 29.595 | 3.01601 | 10.5 |
| 30.925 | 2.88928 | 8.7 |
| 31,122 | 2.87142 | 11.4 |
| 31.858 | 2.80676 | 7.1 |
| 32.930 | 2.71776 | 7.1 |
| 34.332 | 2.60996 | 9.4 |
| 35.049 | 2.55821 | 9.7 |
| 35.760 | 2.50894 | 8.7 |
| 36.247 | 2.47630 | 5.9 |
| 36.652 | 2.44989 | 7.8 |
| 36.862 | 2.43643 | 8.1 |
| 39.276 | 2.29206 | 6.9 |
| 39.721 | 2.26740 | 7.1 |

**Example 15: Crystalline Sitagliptin Citrate**

In a two neck flask sitagliptin free base (2.0 g, 4.91 mmol) and citric acid (0.944 g 4.91 mmol) were dissolved in 10 ml of ethanol and warmed to 80 °C over a water bath for 5 min to obtain clear solution. The solution was seeded with a sitagliptin citrate crystal and kept aside for crystallization. After three days the first crop was filtered and the mother liquor was kept for further crystallization. Since the crystallization was slow 10 ml of ethyl acetate was added as an anti-solvent and the crystallization was continued.

The first crop yielded 1.0 g of sitagliptin citrate having a DSC peak at 163.1 °C ± 2 °C.

The second crop yielded 1.6 g. Both the crops had similar DSC curve.

Table 16: XRPD data of Sitagliptin Citrate

| Angle | d value | Intensity % |
|---|---|---|
| 2-Theta ° | Angstrom | % |
| 5.130 | 17.21167 | 8.8 |
| 7.304 | 12.09286 | 12.9 |
| 8.825 | 10.01219 | 7.0 |
| 9.062 | 9.75052 | 19.0 |
| 10.314 | 8.56970 | 38.5 |
| 12.818 | 6.90076 | 6.7 |
| 14.679 | 6.02996 | 8.0 |
| 15.145 | 5.84531 | 41.5 |
| 15.369 | 5.76065 | 47.9 |
| 16.756 | 5.28673 | 48.2 |
| 16.984 | 5.21618 | 96.6 |
| 17.758 | 4.99063 | 20.3 |
| 18.226 | 4.86359 | 100.0 |
| 18.526 | 4.78543 | 50.6 |
| 19.605 | 4.52453 | 65.8 |
| 20.013 | 4.43312 | 39.9 |
| 20.256 | 4.38058 | 47.4 |
| 21.123 | 4.20270 | 23.7 |
| 22.233 | 3.99521 | 49.9 |
| 22.990 | 3.86533 | 39.6 |
| 23.336 | 3.80882 | 19.6 |
| 23.824 | 3.73197 | 10.2 |
| 24.692 | 3.60265 | 25.2 |
| 25.618 | 3.47449 | 37.1 |
| 26.768 | 3.32773 | 39.0 |
| 27.298 | 3.26433 | 26.4 |
| 27.469 | 3.24439 | 31.8 |
| 29.654 | 3.01014 | 41.2 |
| 30.351 | 2.94260 | 24.7 |
| 32.157 | 2.78136 | 9.9 |
| 32.441 | 2.75763 | 13.2 |
| 33.029 | 2.70988 | 26.8 |
| 34.158 | 2.62283 | 9.9 |
| 36.917 | 2.43288 | 8.3 |

(continued)

| Angle | d value | Intensity % |
|---|---|---|
| 2-Theta ° | Angstrom | % |
| 37.260 | 2.41131 | 9.2 |
| 37.792 | 2.37855 | 8.1 |
| 38.564 | 2:33268 | 9.1 |
| 38.744 | 2.32227 | 9.9 |

**Example 16: Amorphous Sitagliptin Citrate**

In a two neck flask sitagliptin free base (5.0g, 12.28mmol) was dissolved in 20 ml ethyl acetate and warmed at 60 °C to get a clear solution. A solution of anhydrous citric acid (2.36 g, 12.28 mmol) in 20 ml ethyl acetate and 6 ml ethanol was added dropwise over a period of 5 min. A thick white precipitate was observed during the addition. 10 ml of 2-propanol was added and reaction mass was evaporated to dryness to give 7.39 g of sitagliptin citrate. It was confirmed by DSC and XRD that the salt is amorphous.

**Example 17: Sitagliptin Mesylate Form I and Sitagliptin Mesylate Form II**

Sitagliptin free base (6.0 g, 14.742 mmol) was dissolved in 24 ml of ethyl acetate and warmed to 65 °C over a water bath to obtain a clear solution. A solution of methanesulphonic acid (1.4167 g, 14.742 mmol) in 3.0 ml of ethanol was added dropwise over a period of 5 min. The clear solution was kept overnight for crystallization. Optionally the solution can be seeded with crystalline sitagliptin methanesulphonate salt in order to improve crystallisation.
The first crop crystallised after 2 days yielded 2.94 g of sitagliptin mesylate form I having a DSC peak at 125.0 °C ± 2 °C. The ethanol content was 2.97 % indicating that sitagliptin mesylate form I is an ethanol solvate.
A second crop yielded another 0.8 g.
A third crop yielded 3.16 g of sitagliptin mesylate form II having a DSC peak at 161.8 °C ± 2 °C. According to the residual solvent analysis of the third crop no residual ethanol was present.

**Table 17: XRPD data of Sitagliptin Mesylate Form I**

| Angle | d value | Intensity |
|---|---|---|
| 2-Theta ° | Angstrom | % |
| 5.29 | 16.68 | 86.4 |
| 6.42 | 13.76 | 68.7 |
| 6.61 | 13.36 | 100.0 |
| 6.92 | 12.76 | 9.2 |
| 7.64 | 11.56 | 25.1 |
| 9.49 | 9.31 | 8.9 |
| 11.09 | 7.97 | 5.5 |
| 11.43 | 7.74 | 39.5 |
| 12.89 | 6.86 | 8.1 |
| 14.66 | 6.04 | 5.5 |
| 15.34 | 5.77 | 5.6 |
| 15.64 | 5.66 | 15.7 |
| 16.01 | 5.53 | 25.0 |
| 16.44 | 5.39 | 13.4 |
| 17.05 | 5.20 | 30.6 |

(continued)

| Angle | d value | Intensity |
|---|---|---|
| 2-Theta ° | Angstrom | % |
| 17.97 | 4.93 | 33.6 |
| 18.28 | 4.85 | 21.2 |
| 19.21 | 4.62 | 49.4 |
| 19.78 | 4.49 | 9.0 |
| 20.07 | 4.42 | 5.5 |
| 21.44 | 4.14 | 23.0 |
| 21.78 | 4.08 | 34.9 |
| 22.44 | 3.96 | 16.3 |
| 22.64 | 3.92 | 13.1 |
| 23.93 | 3.72 | 34.5 |
| 24.39 | 3.65 | 13.1 |
| 25.92 | 3.43 | 9.8 |
| 26.61 | 3.35 | 8.8 |
| 26.90 | 3.31 | 6.1 |
| 28.69 | 3.11 | 9.8 |
| 29.37 | 3.04 | 9.3 |
| 29.61 | 3.01 | 5.8 |

**Table 18: XRPD data of Sitagliptin Mesylate form II**

| Angle | d value | Intensity |
|---|---|---|
| 2-Theta ° | Angstrom | % |
| 5.43 | 16.27 | 22.2 |
| 8.04 | 11.00 | 14.5 |
| 10.94 | 8.08 | 65.4 |
| 15.04 | 5.88 | 10.6 |
| 15.65 | 5.66 | 7.0 |
| 15.89 | 5.57 | 11.6 |
| 16.12 | 5.49 | 12.2 |
| 17.01 | 5.21 | 33.5 |
| 18.21 | 4.87 | 12.3 |
| 18.89 | 4.69 | 16.6 |
| 19.36 | 4.58 | 69.9 |
| 19.95 | 4.45 | 23.4 |
| 20.43 | 4.34 | 6.0 |
| 21.31 | 4.17 | 44.7 |
| 21.98 | 4.04 | 17.8 |

(continued)

| Angle | d value | Intensity |
|---|---|---|
| 2-Theta ° | Angstrom | % |
| 22.64 | 3.92 | 100.0 |
| 23.79 | 3.74 | 21.9 |
| 24.37 | 3.65 | 34.8 |
| 25.33 | 3.51 | 38.2 |
| 25.84 | 3.45 | 9.6 |
| 27.49 | 3.24 | 11.0 |
| 28.25 | 3.16 | 7.2 |
| 30.51 | 2.93 | 16.1 |
| 32.66 | 2.74 | 6.2 |
| 34.53 | 2.60 | 6.5 |

**Further characterisation of sitagliptin salts**

**Hygroscopicity studies**

The salts of the present invention and the free base were studied under three different humidity conditions (43 % relative humidity, 75 % relative humidity, and 93 % relative humidity). The results are summarized in tables 19-20. The free base, phosphate monohydrate, phosphate, fumarate form I, malate, and maleate did not show much change during the hygroscopicity studies which was also confirmed by DSC of the samples kept at 93 % relative humidity for 2 weeks.

The new sitagliptin sulfate (non solvated; Table 21) showed an increase in water content corresponding to conversion of hemihydrate to dihydrate. DSC also showed an increase in peak due to water uptake.

After two weeks storage at 93% relative humidity the new amorphous sitagliptin citrate (cf. tables 20 and 26) showed a DSC peak at 160.7 ± 2 °C, which is due to an unexpected partial conversion into the respective crystalline form. This conversion process can be used to crystallize amorphous sitagliptin citrate or to increase the crystallinity of sitagliptin citrate.

**Stability Studies**

The stability of the sitagliptin salts of the present invention and the free base were studied by storage of samples at 60 °C for 2 and 4 weeks in closed bottles and storage at 40 °C and 75 % relative humidity (RH) in an open bottle for 12 weeks. The samples were analyzed by HPLC and the content of related substances, which are due to degradation of the sitagliptin, is summarized in tables 22-24.

After storage in a closed bottle for two weeks at 60 °C the following salts were identified as stable compounds: free base, phosphate, phosphate monohydrate, HCl form I, fumarate form I, sulfate (solvated), malate, sulfate (non solvated) and maleate (non solvated) salts. Under these conditions degradation was observed in the case of lactate, succinate, glycolate, citrate and maleate (solvated).

After storage in a closed bottle for four weeks at 60 °C the following salts were identified as stable compounds: free base, phosphate, HCl form I, fumarate form I, sulfate (solvated) and malate. Lactate, succinate form I, glycolate, citrate (amorphous) and maleate showed a degradation.

After storage in an open bottle for twelve weeks at 40 °C and 75 % RH the following salts were identified as stable compounds: free base, phosphate, HCl form I, and fumarate form I. Only lactate shows degradation.

**EXAMPLE FOR PHARMACEUTICAL COMPOSITIONS:**

The crystalline salts of the present invention can be formulated into a tablet by a direct compression process. A 50 mg potency tablet is composed of: 73.58 mg of sitagliptin citrate, 176 mg mannitol, 5 mg of croscarmellose sodium, and 6 mg of magnesium stearate.

The active ingredient, microcrystalline cellulose, and croscarmellose are first blended, and the mixture is then lubricated with magnesium stearate and pressed into tablets.

Table 19: Hygroscopicity studies

| Substance | Base | A: Phosphate | B: HCl I | C: Lactate | D:Fumarate I | E: Sulfate I | F: Succinate I |
|---|---|---|---|---|---|---|---|
| | | | | | | | |
| Mol Formula | $C_{16}H_{15}F_6N_5O$ | Base.$H_3PO_4$ | Base.HCl | B.$C_3H_6O_3$ | B.$C_4H_4O_4$ | B.0.5 $H_2SO_4$ | B.$C_4H_6O_4$ |
| Mol wt of Anhydrous | 407.31 | 505.31 | 443.77 | 497.39 | 523.38 | 456.36 | 525.4 |
| Mol wt of Hydrate | | | 452.77 | | | | |
| DSC of initial sample °C | 118.34 | 213.7 | 95.94, 173.32 | 150.3 | 177.0 | 148.2(major), 197.4(minor) | 126.17 |
| DSC of Hygroscopicity sample (93 % RH, 2 weeks) °C | 118.63 | 212.65 | 113.58, 172.64 | 150.56 # | 177.99 | 92.74 129.43 177.99 198.35 | 63.30 125.12 136.26 # |
| M.P °C | 117.6-118.6 | 210-212 | 169-171 | 149-150 | 172-173 | 147.2-148.1 | |
| Initial water content % | 0.19 | 0.46 | 1.4 | 0.51 | 0.89 | 0.69 | 0.90 |
| Hygroscopicity | | | | | | | |
| 2 weeks 43 % rh | 0.19 | 0.41 | 3.86 | 0.54 | 1.058 | 1.12 | 1.36 |
| 2 weeks 75 % rh | 0.22 | 0.48 | 4.36 | 0.5 | 1.078 | 1.0 | 1.78 |
| 2 weeks 93 % rh * | 0.3 | 0.82 | 4.49 | 28.77 * | 1.168 | 2.81 | 19.58 ** |

* Solid powder changed into liquid state.

\# DSC of hygroscopicity sample kept at 75 % RH for 2 weeks

Table 20: Hygroscopicity studies

| Substance | G: Malate | H: Glycolate | I: Citrate amorphous | J: Mesylate II | K: Mesylate I | L: Maleate I | M: Phosphate |
|---|---|---|---|---|---|---|---|
| | | | | | | | |
| Mol Formula | $B.C_4H_6O_5$ | $B.C_2H_4O_3$ | $B.C_6H_8O_7$ | $B.CH_3SO_3H$ | $B.CH_3SO_3H$ | $B.C_4H_4O_4$ | $Base.H_3PO_4.H_2O$ |
| Mol wt of Anhydrous | 541.4 | 483.36 | 599.43 | 503.42 | 503.42 | 523.38 | 505.31 |
| Mol wt of Hydrate | | | | | | | 523.31 |
| DSC of initial sample °C | 154.38 | 89.1 101.6 | amorphous | 161.8 | 125.0 | 92.51 | 129.9, 212.46 |
| DSC of Hygroscopicity sample (93 % RH, 2 weeks) °C | 154.44 | 68.92<br>95.72 # | 160.7 | 161.48 | 162.81 | 163.46 | 129.05, 212.89 |
| M.P °C | 151-152.3 | 90-92 | < 90 | 156-157 | 121.8-124 | 153-154.8 | |
| Initial water content % | 1.14 | 1.039 | 0.82 | 1.47 | 0.68 | 0.59 | 3.59 |
| Hygroscopicity | | | | | | | |
| 2 weeks 43 % rh | 1.44 | 2.24 ss | 1.19 | 2.58 | 1.13 | -2.34 | 3.69 |
| 2 weeks 75 % rh | 1.58 | β 4.81 | 6.46 ss | 2.94 | 0.49 | β - 7.36 | 3.67 |
| 2 weeks 93 % rh | 1.68 | 24.85 ** | β -2.46 | 3.23 | 0.96 | β - 3.70 | 3.73 |

* = Hygroscopicity data indicates % Equilibrium moisture content
ss = semi solid state of sample
β = Solid sample first changed into liquid and again it solidified
# DSC of hygroscopicity sample kept at 75 % RH for 2 weeks

Table 21: Hygroscopicity studies

| Substance | N: Mesylate II | O: Sulfate II | P: Maleate II | Q: HCl II | R: Citrate crystalline | S: Succinate III | Fumarate II |
|---|---|---|---|---|---|---|---|
| | | | | | | | |
| Mol Formula | $B.CH_3SO_3H$ | $B.0.5\ H_2SO_4$ | $B.C_4H_4O_4$ | Base.HCl | $B.C_6H_8O_7$ | $B.C_4H_6O_4$ | $B.C_4H_4O_4$ |
| Mol wt of Anhydrous | 503.42 | 456.36 | 523.38 | 443.77 | 599.43 | 525.4 | 523.38 |
| Mol wt of Hydrate | | | | | | | |
| DSC of initial sample °C | 159.51 | 101.14<br>175.9 (major)<br>195.9 (minor) | 159.5 | 202.3 | 163.1 | 167.8 | 188.36 |
| DSC of Hygroscopicity sample (93 % RH, 2 weeks) °C | 159.85 | 91.4<br>175.1 | 161.12 | 82.5<br>170.6<br>204.37 | 164.74 | 168.60 | 190.84 |
| M.P °C | 156-157 | 170-173.8 | 153-154.8 | 203 | 163.2 | 167.8 | 189 |
| Initial water content % | 2.08 | 1.88 | 0.13 | 0.27 | 0.47 | 0.18 | 0.28 |
| Hygroscopicity | | | | | | | |
| 2 weeks 43 % rh | 2.89 | 2.59 | 0.016 | 0.32 | 0.82 | 0.20 | 0.23 |
| 2 weeks 75 % rh | 3.37 | 7.1 | -0.044 | 0.44 | 0.29 | 0.24 | 0.30 |
| 2 weeks 93 % rh | 4.04 | 7.33 | -0.071 | 0.83 | 0.42 | 0.67 | 0.22 |

Table 22: Stability studies

| Substance | Base | A: Phosphate | B: HCl I | C: Lactate | D: Fumarate I | E: Sulfate I | F: Succinate I |
|---|---|---|---|---|---|---|---|
| Stability | | | | | | | |
| Initial Related Substances % | 0.36 | 0.39 | 0.04 | 0.16 | 0 | 0.08 | 0.14 |
| 2 W 60 °C closed | 0.37 | 0.50 | 0.01 | 0.43 | 0.01 | 0.08 | 0.30 |
| 2 W 40 °C175 % RH open | 0.27 | 0.21 | 0.05 | 0.34 | 0.01 | 0.10 | 0.13 |
| 4 w 40 °C/75 % RH closed | 0.46 | 0.31 | 0.01 | 0.28 | 0.07 | 0.02 | 0.29 |
| 4 w 40 °C/75 % RH open | 0.39 | 0.16 | 0.01 | 0.33 | 0.06 | 0.06 | 0.25 |
| 4 w 60 °C Closed bottle | 0.46 | 0.38 | 0.01 | 0.49 | 0.03 | 0.02 | 0.58 |
| 12 weeks 40 °C/75 % RH open bottle | 0.39 | 0.37 | 0.15 | 0.59 | 0.11 | 0.08 | 0.16 |
| 12 weeks 40 °C/75 % RH closed bottle | 0.52 | 0.57 | 0.14 | 0.56 | 0.13 | 0.03 | 0.23 |

Table 23: Stability studies

| Substance | G: Malate | H: Glycolate | I: Citrate amorphous | L: Maleate I | M: Phosphate H2O |
|---|---|---|---|---|---|
| Stability | | | | | |
| Initial Related Substances % | 0.01 | 0.05 | 0.66 | 0.22 | 0.08 |
| 2 W 60 °C closed | 0.05 | 1.81 | 4.09 | 1.36 | 0.12 |
| 2 W 40 °C/75 % RH open | 0.06 | 0.24 | 1.52 | 0.32 | 0.10 |
| 4 w 40 °C/75 % RH closed | 0.10 | 0.49 | 2.68 | 0.49 | 0.08 |
| 4 w 40 °C/75 % RH open | 0.08 | 0.61 | 2.38 | 0.79 | 0.09 |
| 4 w 60 °C Closed bottle | 0.09 | 4.06 | 17.71 | 1.21 | 0.06 |
| 12 weeks 40 °C/75 % RH open bottle | 0.09 | 0.99 | 3.86 | 2.05 | 0.12 |
| 12 weeks 40 °C/75 % RH closed bottle | 0.08 | 1.09 | 5.92 | 1.65 | 0.09 |

Table 24: Stability studies

| Substance | N: Mesylate II | O: Sulfate II | P: Maleate II | Q: HCl II | R: Citrate crystalline | S: Succinate III | Fumarate II |
|---|---|---|---|---|---|---|---|
| Stability | | | | | | | |

(continued)

| Substance | N: Mesylate II | O: Sulfate II | P: Maleate II | Q: HCl II | R: Citrate crystalline | S: Succinate III | Fumarate I |
|---|---|---|---|---|---|---|---|
| Initial Related Substances % | 0.11 | 0.20 | 0.12 | 0.20 | 0.14 | 0.29 | 0.02 |
| 2 W 60 °C closed | 0.16 | 0.20 | 0.21 | 0.23 | 0.27 | 0.38 | 0.10 |
| 2 W 40 °C/75 % RH open | 0.16 | 0.23 | 0.20 | 0.36 | 0.18 | 0.31 | 0.08 |
| 4 w 40 °C/75 % RH closed | 0.08 | 0.24 | 0.15 | 0.30 | 0.20 | 0.32 | 0.15 |
| 4 w 40 °C/75% RH open | 0.08 | 0.36 | 0.15 | 0.53 | 0.21 | 0.35 | 0.14 |
| 4 w 60 °C Closed bottle | 0.12 | 0.31 | 0.24 | 0.30 | 0.40 | 0.44 | 0.27 |
| 12 weeks 40 °C/75 % RH open bottle | 0.11 | 0.46 | 0.29 | 0.44 | 0.52 | 0.45 | 0.14 |
| 12 weeks 40 °C/75 % RH closed bottle | 0.07 | 0.25 | 0.12 | 0.34 | 0.37 | 0.38 | 0.10 |

Table 25: DSC characterisation

| Substance | Base | A: Phosphate | B: HCl I | | C: Lactate | D: Fumarate I | E: Sulfate I | | | | F: Succinate I |
|---|---|---|---|---|---|---|---|---|---|---|---|
| Code | 006/014 | 006/054 | 006/056 | | 006/058 | 006/076 | 006/122 | | | | 006/166-1 |
| Mol Formula | $C_{16}H_{15}F_5N_5O$ | Base.$H_3PO_4$ | Base.HCl | | B.$C_3H_6O_3$ | B.$C_4H_4O_4$ | B.0.5 $H_2SO_4$ | | | | B.$C_4H_6O_4$ |
| Mol wt of Anhydrous | 407.31 | 505.31 | 443.77 | | 497.39 | 523.38 | 456.36 | | | | 525.4 |
| Mol wt of Hydrate | | | 452.77 | | | | | | | | |
| M.P °C | 117.6 - 118.6 | 210-212 | 169-171 | | 149-150 | 172-173 | 147.2-148.1 | | | | 119-120.6 |
| DSC of initial sample | | | | | | | | | | | |
| Onset temperature °C | 115.81 | 209.56 | 74.78 | 166.36 | 147.81 | 174.52 | 136.29 | 168 | 174.6 | 195.70 | 118.17 |
| Endset temperature °C | 120.80 | 215.81 | 100.92 | 178.77 | 153.13 | 178.76 | 154.03 | 173.8 | 178.7 | 198.93 | 131.73 |
| Peak temperature °C | 118.34 | 213.69 | 95.94 | 173.22 | 150.34 | 177.03 | 148.21 | 171.4 | 176.1 | 197.36 | 126.17 |
| Enthalapy J/g | -89.60 | -147.54 | -20.31 | -53.99 | -82.05 | -78.52 | -50.57 | -0.54 | -0.16 | -3.84 | -57.58 |
| DSC of samples kept at 93 % RH for 2 weeks | | | | | # | | | | | | |
| Onset temperature °C | 116.10 | 208.43 | 88.44 | 166.78 | 148.68 | 175.92 | 72.62 | 120.09 | 173.0 | 196.7 | 118.8 |
| Endset temperature °C | 120.88 | 215.20 | 117.30 | 176.10 | 152.04 | 180.04 | 99.05 | 134.34 | 181.3 | 200.0 | 129.4 |
| Peak temperature °C | 118.63 | 212.65 | 113.58 | 172.64 | 150.56 | 177.99 | 92.7 | 129.4 | 178 | 198.4 | 125.1 |
| Enthatapy J/g | -94.78 | -102.66 | -63.70 | -54.51 | -81.95 | -84.18 | -65.9 | -15.7 | - 37.86 | -7.17 | -64.9 |

Table 26: DSC characterisation

| Substance | G: Malate | H: Glycolate | | I: Citrate amorphous | J: Mesylate II | K: Mesylate I | L: Maleate I | M: Phosphate | |
|---|---|---|---|---|---|---|---|---|---|
| Code | 007.024 | 007/22A | | 006/180 | 007/004-I | 007/004-III | 007/048 | ST07122003-02 | |
| Mol Formula | $B.C_4H_6O_5$ | $B.C_2H_4O_3$ | | $B.C_6H_8O_7$ | $B.CH_3SO_3H$ | $B.CH_3SO_3H$ | $B.C_4H_4O_4$ | $Base.H_3PO_4.H_2O$ | |
| Mol wt of Anhydrous | 541.4 | 483.36 | | 599.43 | 503.42 | 503.42 | 523.38 | 505.31 | |
| Mol wt of Hydrate | | | | | | | | 523.31 | |
| M.P °C | 151-152.3 | 90-92 | | <90 | 157.8-158.9 | 121.8-124 | 90-91 (not complete) | | |
| DSC of initial sample °C<br>Onset temperature °C | 151.32 | 93.99 | | amorphous | 154.29 | 115.14 | 85.81 | 116.12 | 210.25 |
| Endset temperature °C | 157.05 | 105.79 | | | 165.73 | 134.14 | 96.77 | 136.19 | 214.31 |
| Peak temperature °C | 154.38 | 101.62 | | | 161.84 | 125.04 | 92.51 | 129.87 | 212.46 |
| Enthalapy J/g | -82.61 | -15.70 | | | -54.19 | -40.15 | -69.71 | -102.12 | -107.86 |
| DSC of samples kept at 93 % RH for 2 weeks<br>Onset temperature °C | 151.67 | #<br>48.5 | 89.55 | 152.60 | 153.93 | 153.91 | 157.68 | 116.31 | 210.36 |
| Endset temperature °C | 157.19 | 80.15 | 100.67 | 165.88 | 165.66 | 168.45 | 166.73 | 135.15 | 214.62 |
| Peak temperature °C | 154.44 | 68.92 | 95.75 | 160.70 | 161.48 | 162.81 | 163.46 | 129.05 | 212.89 |
| Enthalapy J/g | -87.41 | -37.8 | -12.49 | -53.67 | -51.74 | -51.53 | -36.98 | -102.62 | -101.76 |
| # DSC of sample kept at 75 % RH for 2 weeks | | | | | | | | | |

Table 27: DSC characterisation

| Substance | N: Mesylate | O: Sulfate | | | P: Maleate | Q: HCl II | | | R: Citrate crystalline | S: Succinate III | Fumarate II |
|---|---|---|---|---|---|---|---|---|---|---|---|
| Code | 010/040 | 007/106 | | | 010/046 | 010/060 | | | 010/050 | 007/070 | 006/082 |
| Mol Formula | $B.CH_3SO_3H$ | $B.0.5\ H_2SO_4$ | | | $B.C_4H_4O_4$ | Base.HCl | | | $B.C_6H_8O_7$ | $B.C_4H_6O_4$ | $B.C_4H_4O_4$ |
| Mol wt of Anhydrous | 503.42 | 456.36 | | | 523.38 | 443.77 | | | 599.43 | 525.4 | 523.38 |
| Mol wt of Hydrate | | | | | | | | | | | |
| M.P °C | 156-157 | 170-173.8 | | | 153-154.8 | 203 | | | 163.2 | 167.8 | 189 |
| DSC of initial sample °C<br>Onset temperature °C | 152.78 | 68.30 | 169.52 | 194.61 | 152.22 | 195.81 | | | 157.34 | 165.93 | 185.06 |
| Endset temperature °C | 162.52 | 115.57 | 180.44 | 197.82 | 163.02 | 205.28 | | | 165.81 | 169.74 | 190.73 |
| Peak temperature °C | 159.51 | 101.14 | 175.89 | 195.93 | 159.45 | 202.30 | | | 163.12 | 167.77 | 188.36 |
| Enthalapy J/g | -49.49 | -7.87 | -37.73 | -2.67 | -70.10 | -77.99 | | | -54.38 | -102.96 | -58.91 |
| DSC of samples kept at 93 % RH for 2 weeks<br>Onset temperature °C | 153.83 | 74.40 | 170.83 | | 152.88 | 77.18 | 167.48 | 199.65 | 160.02 | 166.93 | 187.50 |
| Endset temperature °C | 163.16 | 96.20 | 177.88 | | 164.17 | 89.48 | 173.46 | 207.16 | 167.10 | 170.43 | 192.90 |
| Peak temperature °C | 159.85 | 91.39 | 175.13 | | 161.12 | 82.48 | 170.63 | 204.37 | 164.74 | 168.60 | 190.84 |
| Enthalapy J/g | -49.15 | -35.13 | -23.58 | | -76.55 | -0.61 | -0.18 | -81.83 | -67.15 | -83.49 | -91.17 |

Table 28: Dissolution results of salts (Done on 1 capsule each)

| | Phosphate H2O Ref | HCl II | Fumarate I | Fumarate II | Citrate | Malate |
|---|---|---|---|---|---|---|
| | ST07122003-02 | 010/60 | 006/76 | 006/82 | 010/050 | 007/24 |
| Dissolution in capsule in pH 6.8 buffer at 50 rpm at 10 min (% release as free base) | 86.65 | 90.13 | 90.34 | 94.55 | 62.4 | 84.72 |
| Dissolution in capsule in pH 6.8 buffer at 50 rpm at 30 min (% Release as free base) | 91.12 | 91.21 | 91.76 | 97.79 | 89.47 | 90.94 |
| Dissolution in capsule in water medium at 50 rpm at 10 min (% release as free base) | 46.63 | | | | 75.89 | 90.22 |
| Dissolution in capsule in water medium at 50 rpm at 30 min (% release as free base) | 96.30 | | | | 94.23 | 98.89 |
| Dissolution in capsule in phosphate buffer pH 7.4 medium at 50 rpm at 10 min (% release as free base) | 80.81 | 93.63 | | 99.51 | | |
| Dissolution in capsule in phosphate buffer pH 7.4 medium at 50 rpm at 30 min (% release as free base) | 99.18 | 99.67 | | >100 | | |
| Dissolution in capsule in acetate buffer pH 4.5 medium at 50 rpm at 10 min (% release as free base) | 48.08 | 93.89 | | 98.99 | | |
| Dissolution in capsule in acetate buffer pH 4.5 medium at 50 rpm at 30 min (% release as free base) | 98.48 | 100 | | >100 | | |

Table 29: Evaluation of different Sitagliptin salts for processability

| | D: Fumarate I | P: Maleate II | S: Succinate III | G: Malate | O: Sulfate II | R: Citrate crystalline | M: Phosphate |
|---|---|---|---|---|---|---|---|
| **Appearance** | White to off-white powderwith coarse particles. | White to off-white powder with coarse, hard particles inside. Electrostatic character! | White to off-white powder without coarse particles. Sticks to cylinder glass. | White to off white powder with lots of coarse particles which are easy to crush. | White to off-white powder with big, coarse and hard particles. | White to off-white powder with heterogeneous, small particles which are easy to crush. | White to off-white powder, fine powder, few agglomerates. |
| **Apparent Volume** | | | | | | | |
| $V_0$ [ml] | 64 | 70 | 60 | 52 | 40 | 30 | 190 |
| $V_{10}$ [ml] | 62 | 70 | 58 | 50 | 40 | 30 | 185 |
| $V_{500}$ [ml] | 58 | 62 | 50 | 46 | 38 | 26 | 175 |
| $V_{1250}$ [ml] | 57 | 60 | 48 | 46 | 38 | 26 | 174 |
| **Poured density, $m/V_0$ [g/ml]** | 0.363 | 0.200 | 0.370 | 0.400 | 0.483 | 0.253 | 0.52 |
| **Tapped density, $m/V_{1250}$ [g/ml]** | 0.407 | 0.233 | 0.463 | 0.452 | 0.508 | 0.292 | 0.57 |
| **Sticking tendency** | Low, greasy | Low, greasy | High | Low | Very low | Very low | None |
| **Flowability** | Good | Ok | Good | Ok | Good | Good | Good |

## Claims

1. Crystalline salt of sitagliptin with malic acid, having in the XRPD pattern (determined by using Cu Kα radiation) characteristic peaks at 2 theta angles of 15.9 ± 0.2°, 18.0 ± 2° and 23.2 ± 0.2°.

2. The crystalline salt of sitagliptin according to claim 1 having an XRPD pattern as described in Figure 5.

3. Process for the preparation of a crystalline salt of sitagliptin of any one of claims 1 or 2, comprising the steps of

   a. dissolving or suspending sitagliptin in a suitable solvent,
   b. adding malic acid to the solution or suspension,
   c. inducing crystallization of the salt, and
   d. recovering the crystalline salt.

4. The process according to claim 3, wherein the solvent is selected from the group consisting of methanol, ethanol, n-propanol, n-butanol, isopropanol, ethylacetat and any mixture thereof.

5. Use of a crystalline salt of sitagliptin according to any one of claims 1 or 2 for preparing a pharmaceutical composition.

6. Pharmaceutical composition comprising a crystalline salt of sitagliptin according to any one of claims 1 or 2.

7. The pharmaceutical composition according to claim 6, further comprising a second active pharmaceutical ingredient.

8. The pharmaceutical composition according to claim 7, wherein the second active pharmaceutical ingredient is a biguanide or metformin, preferably metformin hydrochloride.

9. Crystalline salt of sitagliptin according to any one of claims 1 or 2 for use in the treatment or prevention of non insulin dependent diabetes mellitus, obesity, insulin resistance, syndrome X and type 2 diabetes.

## Patentansprüche

1. Kristallines Salz von Sitagliptin mit Äpfelsäure, das im Röntgenpulverdiffraktogramm (bestimmt durch die Verwendung von Cu Kα Strahlung) charakteristische Peaks bei 2-Theta Winkeln von 15,9 ± 0.2°, 18.0 ± 2° und 23.2 ± 0.2° hat.

2. Das kristalline Salz von Sitagliptin nach Anspruch 1 mit einem Röntgenpulverdiffraktogramm, das im Wesentlichen wie in der Abbildung 5 beschrieben ist.

3. Verfahren zur Herstellung eines kristallinen Salzes von Sitagliptin nach Anspruch 1 oder 2, umfassend die Schritte

   a. der Auflösung oder des Suspendierens von Sitagliptin in einem geeigneten Lösungsmittel,
   b. der Addition von Äpfelsäure zu der Lösung oder Suspension,
   c. des Induzierens der Kristallisation des Salzes und
   d. der Gewinnung des kristallinen Salzes.

4. Das Verfahren nach Anspruch 3, wobei das Lösungsmittel ausgewählt ist aus der Gruppe bestehend aus Methanol, Ethanol, n-Propanol, n-Butanol, Isopropanol, Ethylacetat und einer beliebigen Mischung davon.

5. Verwendung eines kristallinen Salzes von Sitagliptin nach einem der Ansprüche 1 oder 2 zur Herstellung einer pharmazeutischen Zusammensetzung.

6. Pharmazeutische Zusammensetzung umfassend ein kristallines Salz von Sitagliptin nach einem der Ansprüche 1 oder 2.

7. Die pharmazeutische Zusammensetzung nach Anspruch 6, ferner umfassend einen zweiten pharmazeutischen Wirkstoff.

8. Die pharmazeutische Zusammensetzung nach Anspruch 7, wobei der zweite pharmazeutische Wirkstoff ein Biguanid oder ein Metformin, vorzugsweise Metformin Hydrochlorid, ist.

9. Kristallines Salz von Sitagliptin nach einem der Ansprüche 1 oder 2 zur Verwendung in der Behandlung oder Prophylaxe von nicht insulinabhängigem Diabetes mellitus, Fettleibigkeit, Insulinresistenz, Syndrom X und Typ 2 Diabetes.

**Revendications**

1. Sel cristallin de sitagliptine avec de l'acide malique, possédant, dans le diagramme XRPD (déterminé en utilisant un rayonnement de Cu K$\alpha$), des pics caractéristiques à des angles 2 thêta de 15,9 $\pm$ 0,2°, 18,0 $\pm$ 2° et 23,2 $\pm$ 0,2°.

2. Le sel cristallin de sitagliptine selon la revendication 1, ayant un diagramme XRPD sensiblement tel qu'illustré sur la figure 5.

3. Processus de préparation d'un sel cristallin de sitagliptine selon les revendications 1 ou 2, comprenant les étapes consistant à

   a. dissoudre ou suspendre la sitagliptine dans un solvant adapté,
   b. ajouter de l'acide malique à la solution ou la suspension,
   c. induire une cristallisation du sel et
   d. récupérer le sel cristallin.

4. Le processus selon la revendication 3, dans lequel le solvant est choisi dans le groupe composé du méthanol, de l'éthanol, du n-propanol, du n-butanol, de l'isopropanol, de l'acétate d'éthyle et d'un mélange quelconque de ces éléments.

5. Utilisation d'un sel cristallin de sitagliptine selon les revendications 1 ou 2 pour la préparation d'une composition pharmaceutique.

6. Composition pharmaceutique comprenant un sel cristallin de sitagliptine selon les revendications 1 ou 2.

7. La composition pharmaceutique selon la revendication 6, comprenant en outre un second ingrédient pharmaceutique actif.

8. La composition pharmaceutique selon la revendication 7, dans lequel le second ingrédient pharmaceutique actif est du biguanide ou de la metformine, de préférence du chlorhydrate de metformine.

9. Sel cristallin de sitagliptine selon les revendications 1 ou 2, à utiliser dans le traitement ou la prévention du diabète sucré non-insulinodépendant, l'obésité, la résistance à l'insuline, le syndrome X et le diabète de type 2.

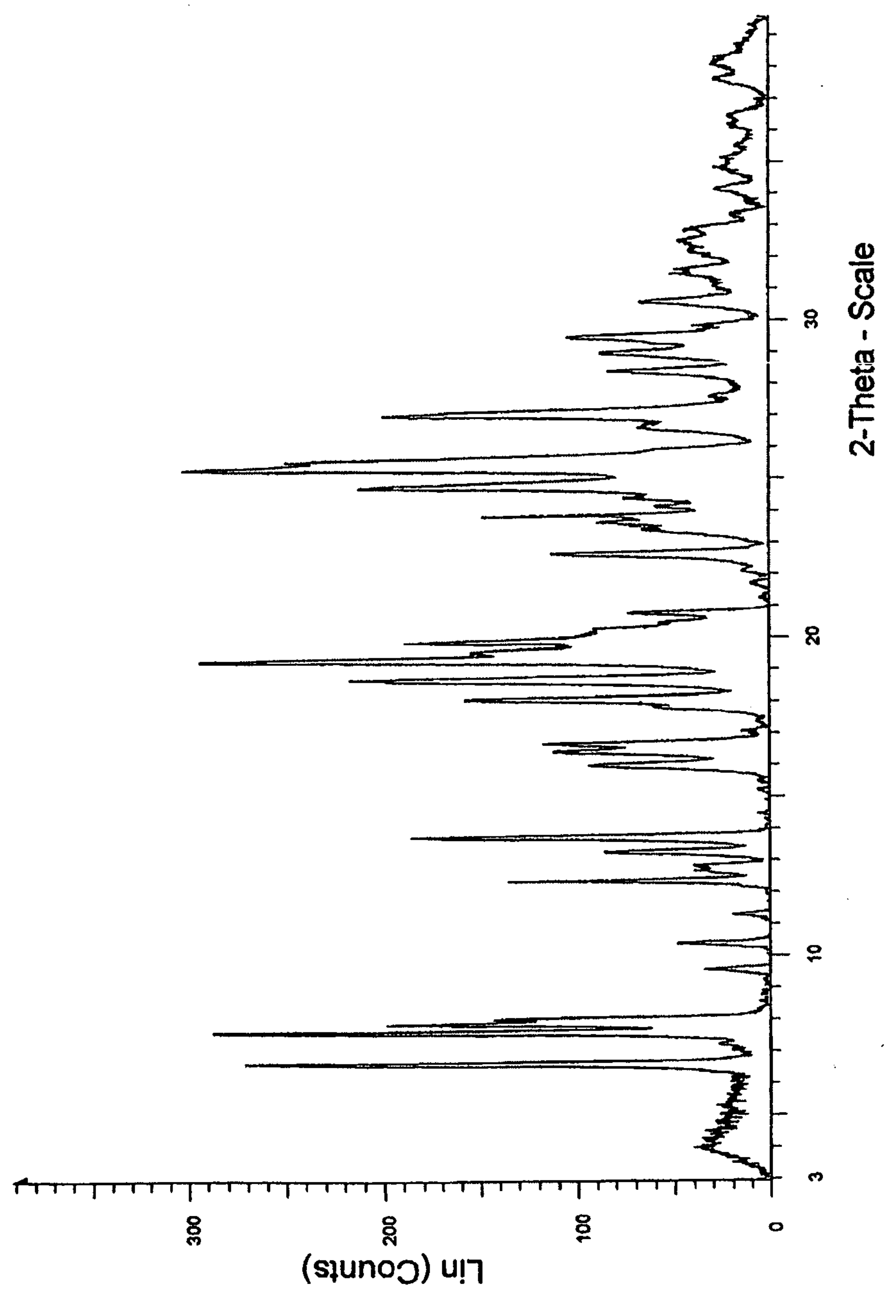


**Fig. 1 XRPD-pattern of sitagliptin HCl Form I**

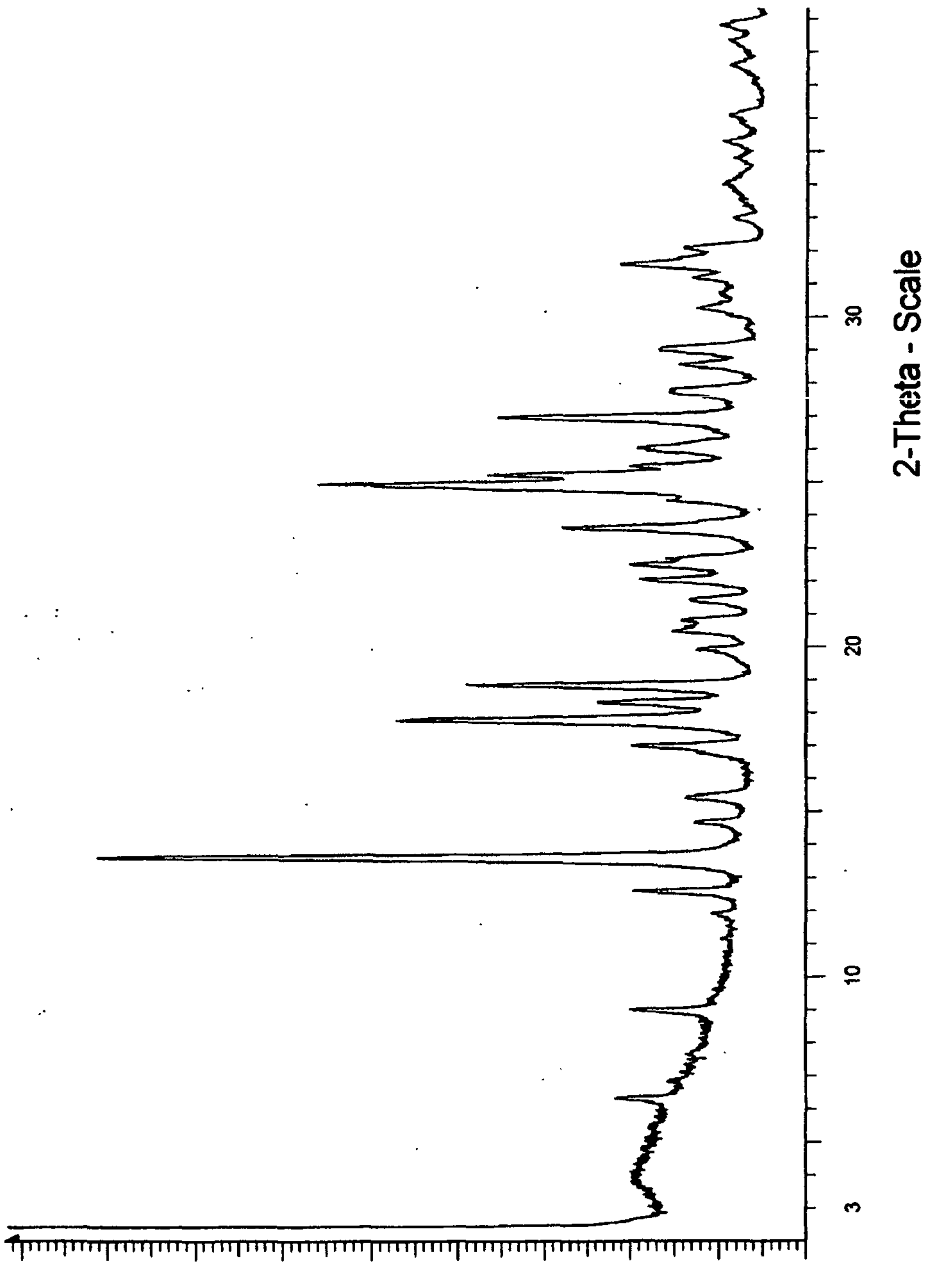


**Fig. 2 XRPD-pattern of sitagliptin HCl Form II**

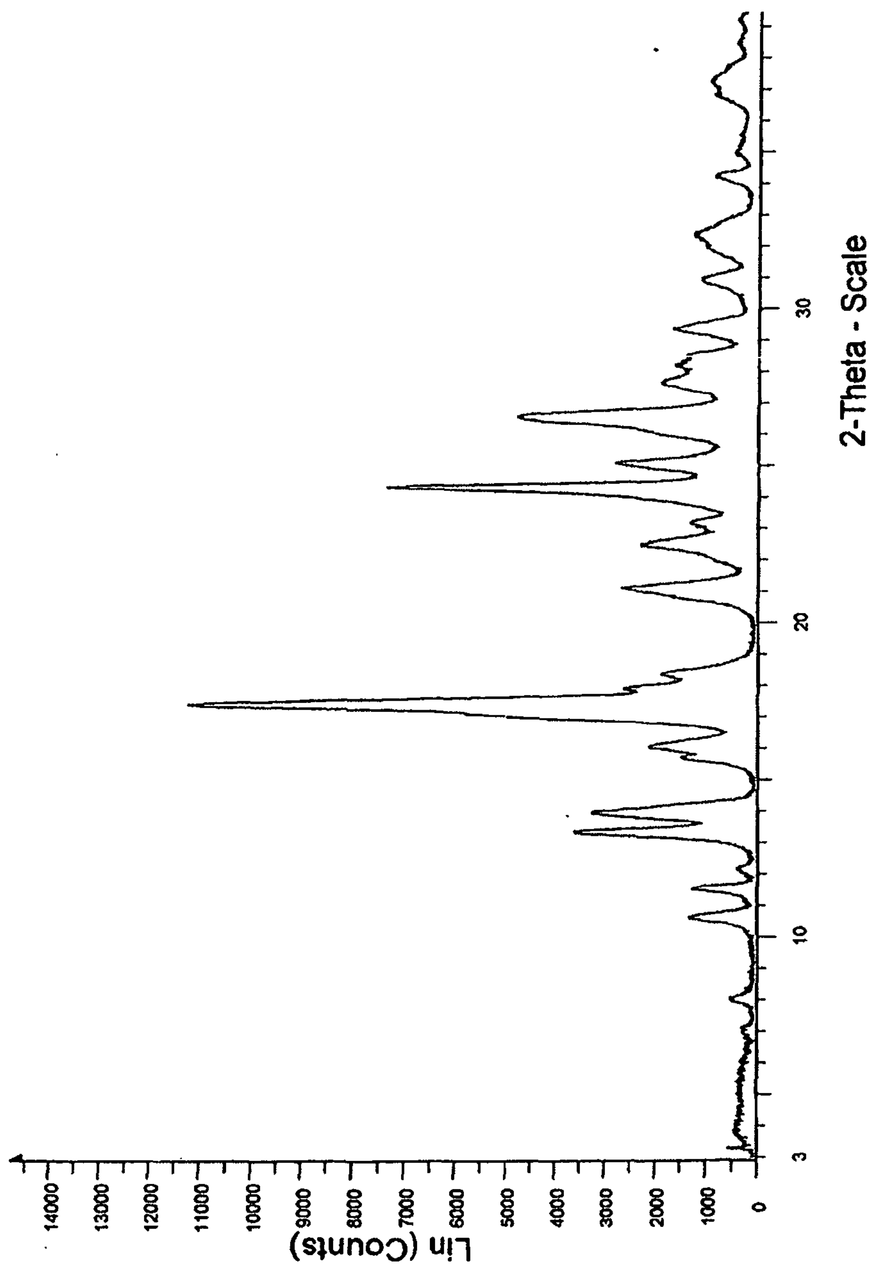


**Fig. 3 XRPD-pattern of sitagliptin fumarate Form I**

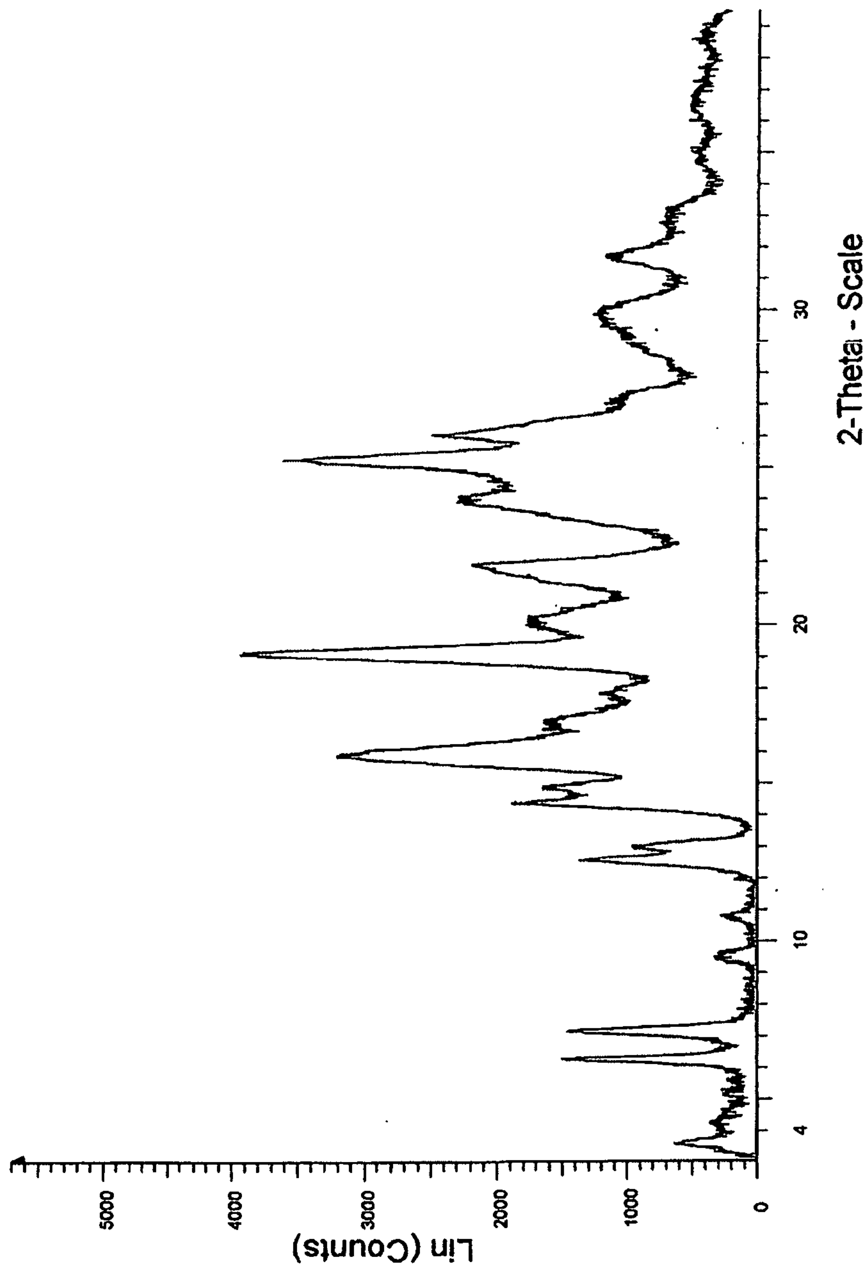


**Fig. 4 XRPD-pattern of sitagliptin fumarate Form II**

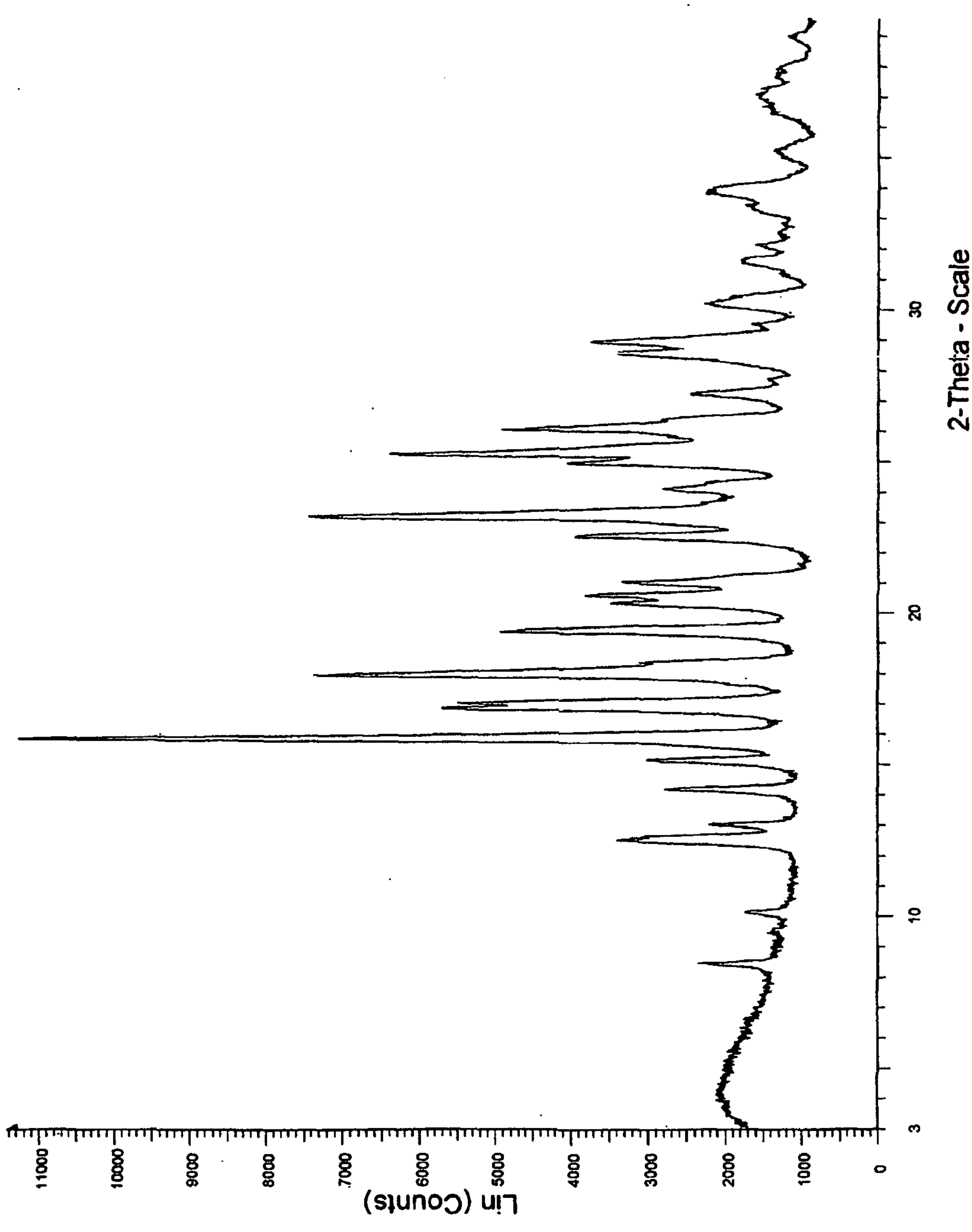


**Fig. 5 XRPD-pattern of sitagliptin malate**

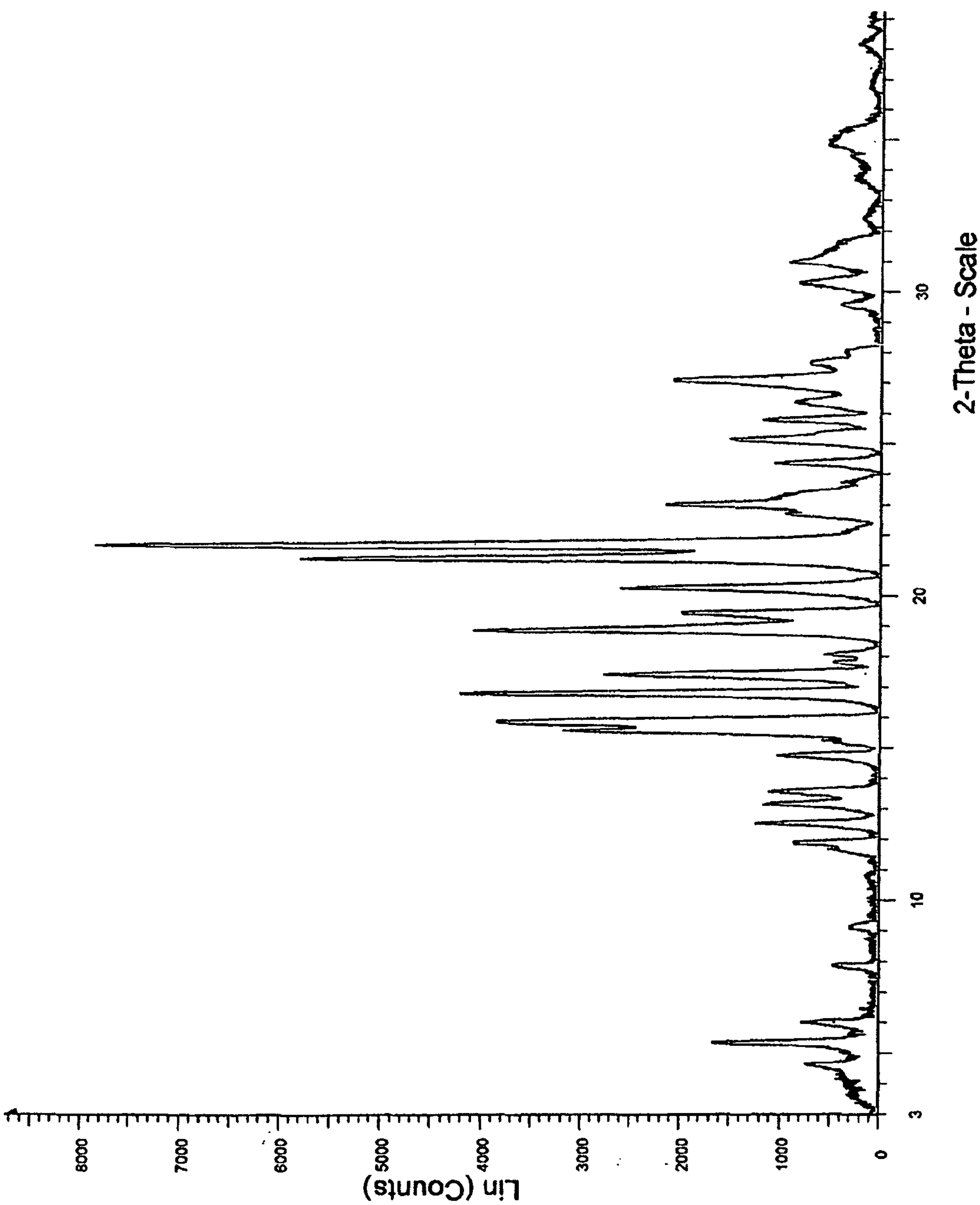


Fig. 6 XRPD-pattern of sitagliptin sulphate Form I

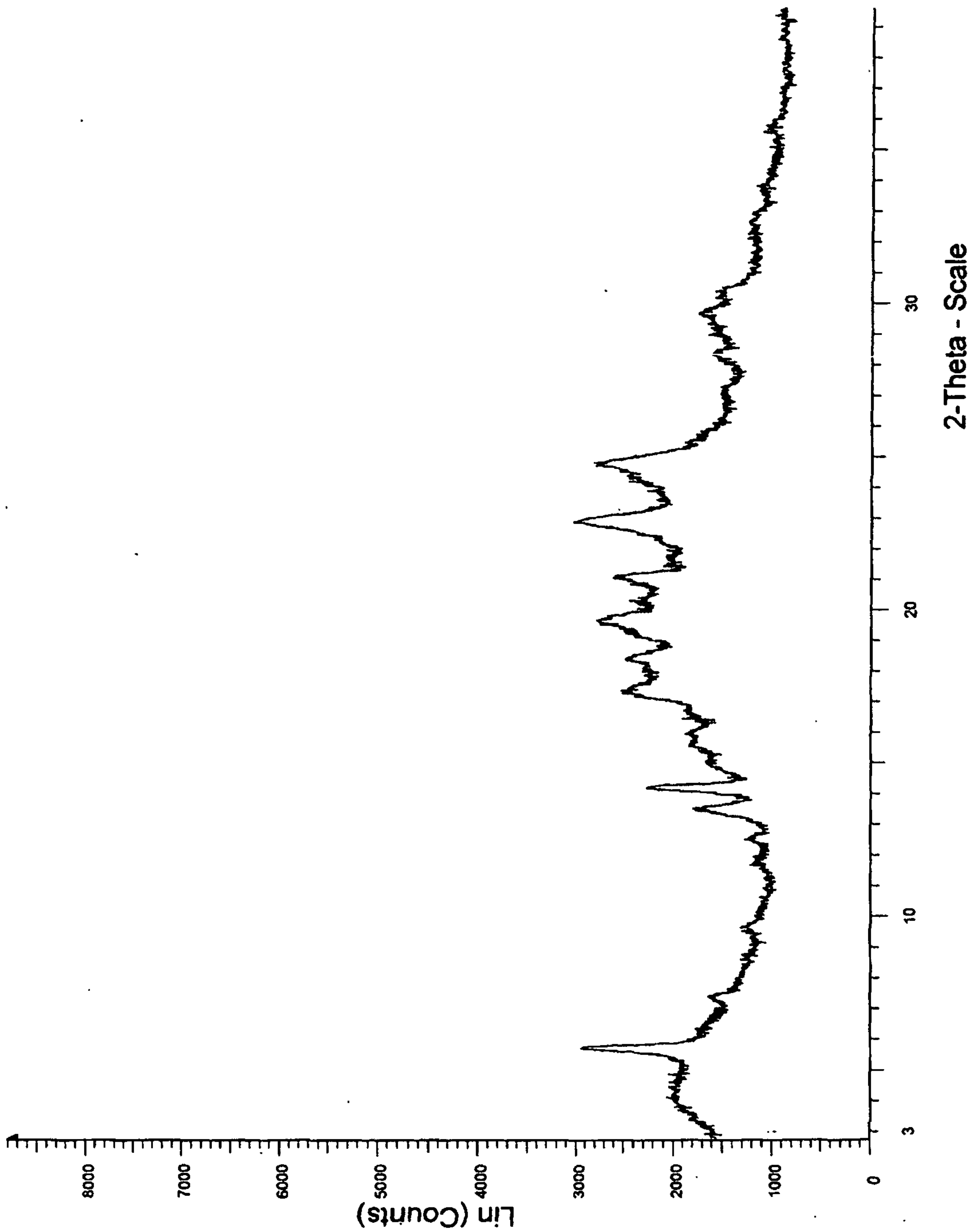


**Fig. 7 XRPD-pattern of sitagliptin sulphate Form II**

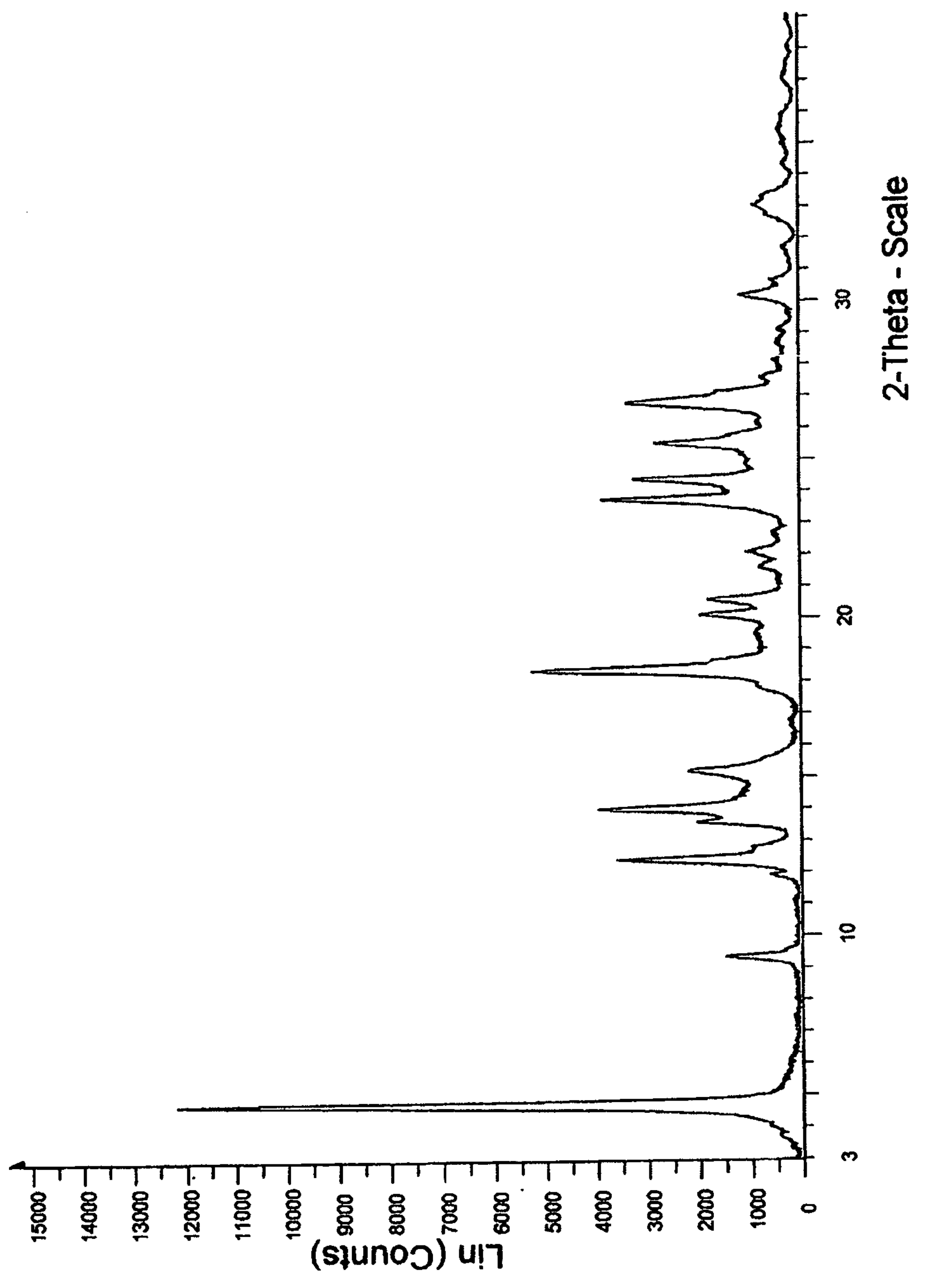


**Fig. 8 XRPD-pattern of sitagliptin phosphate**

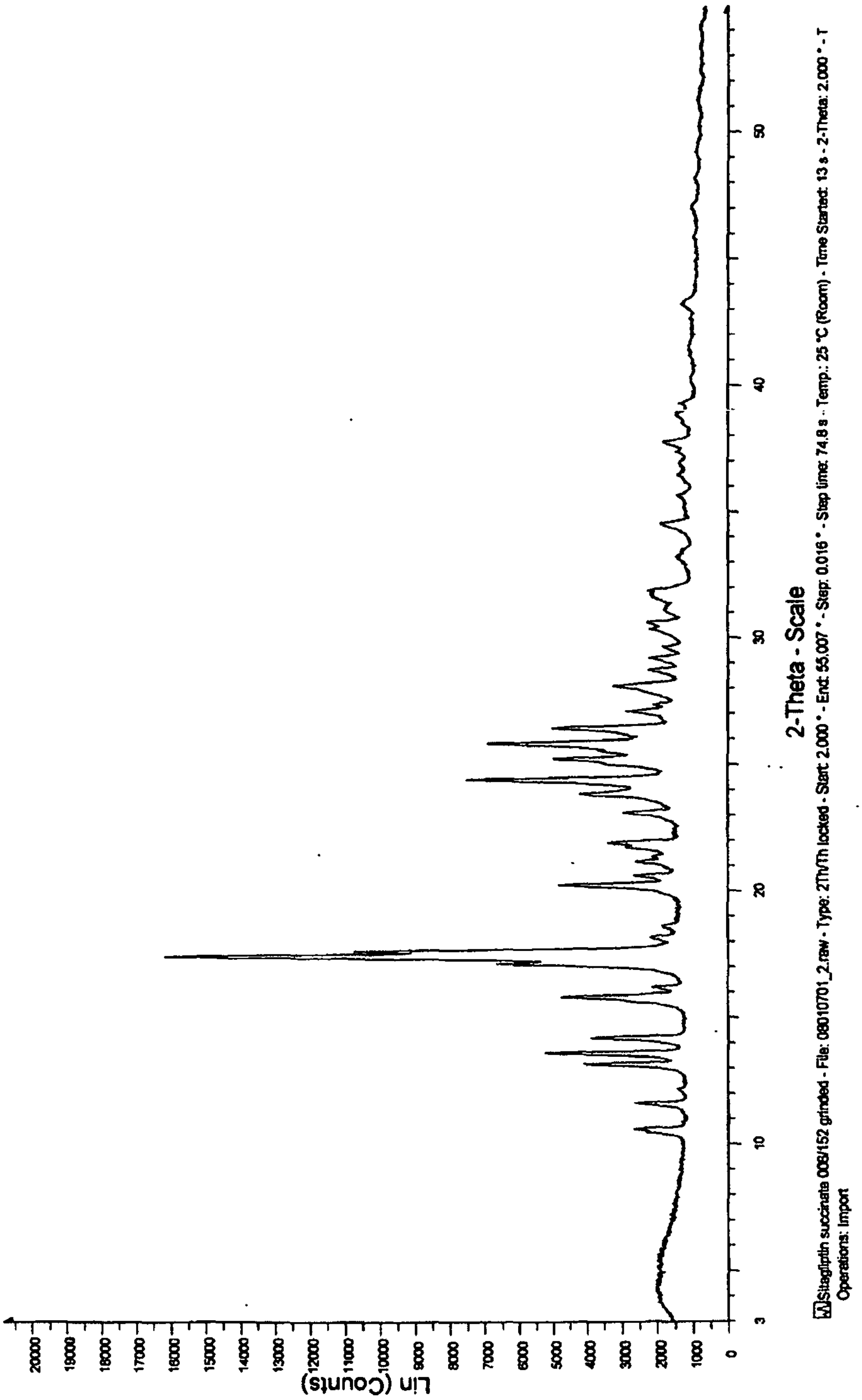


**Fig. 9 XRPD-pattern of sitagliptin succinate Form I**

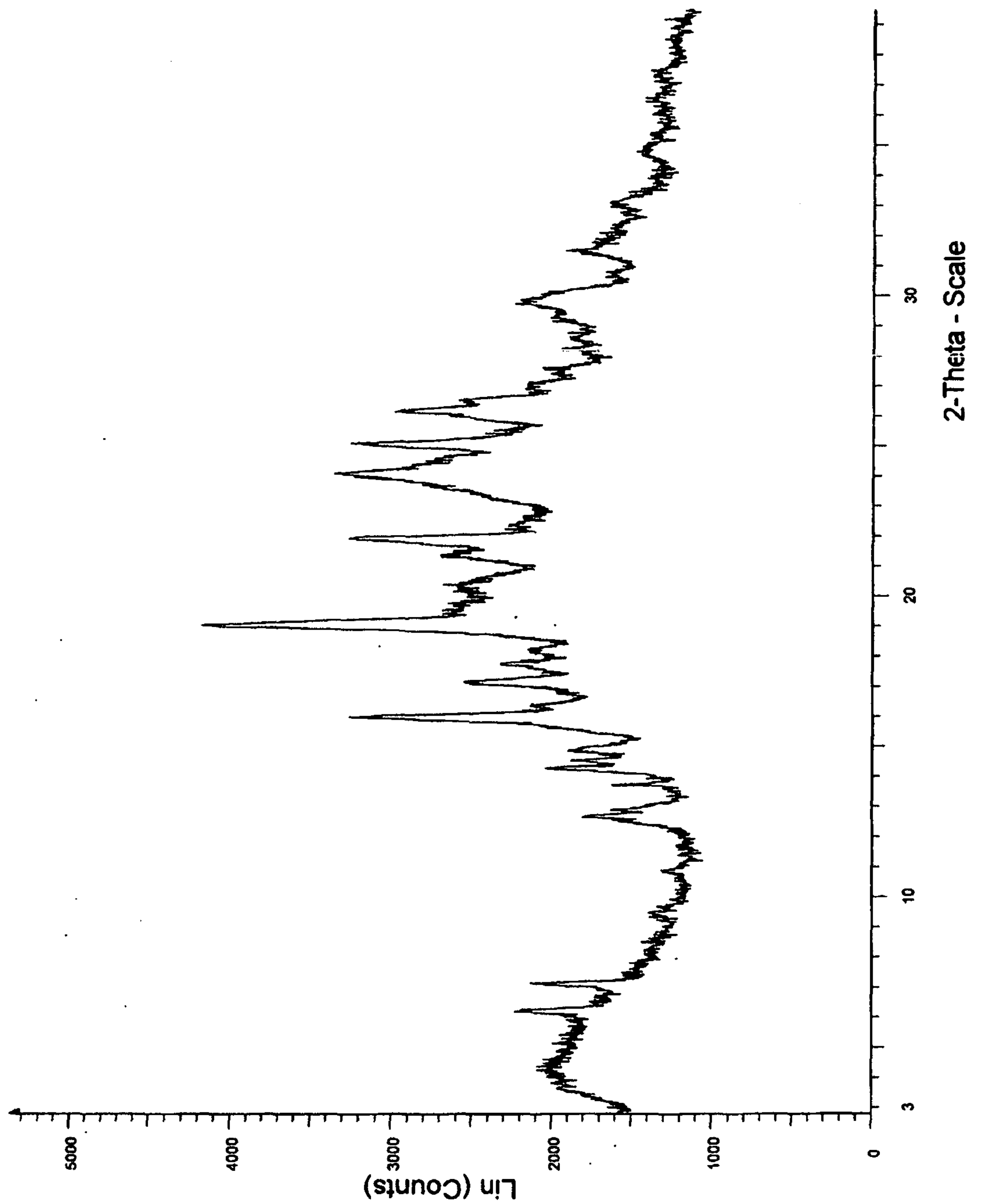


**Fig. 10 XRPD-pattern of sitagliptin succinate Form II**

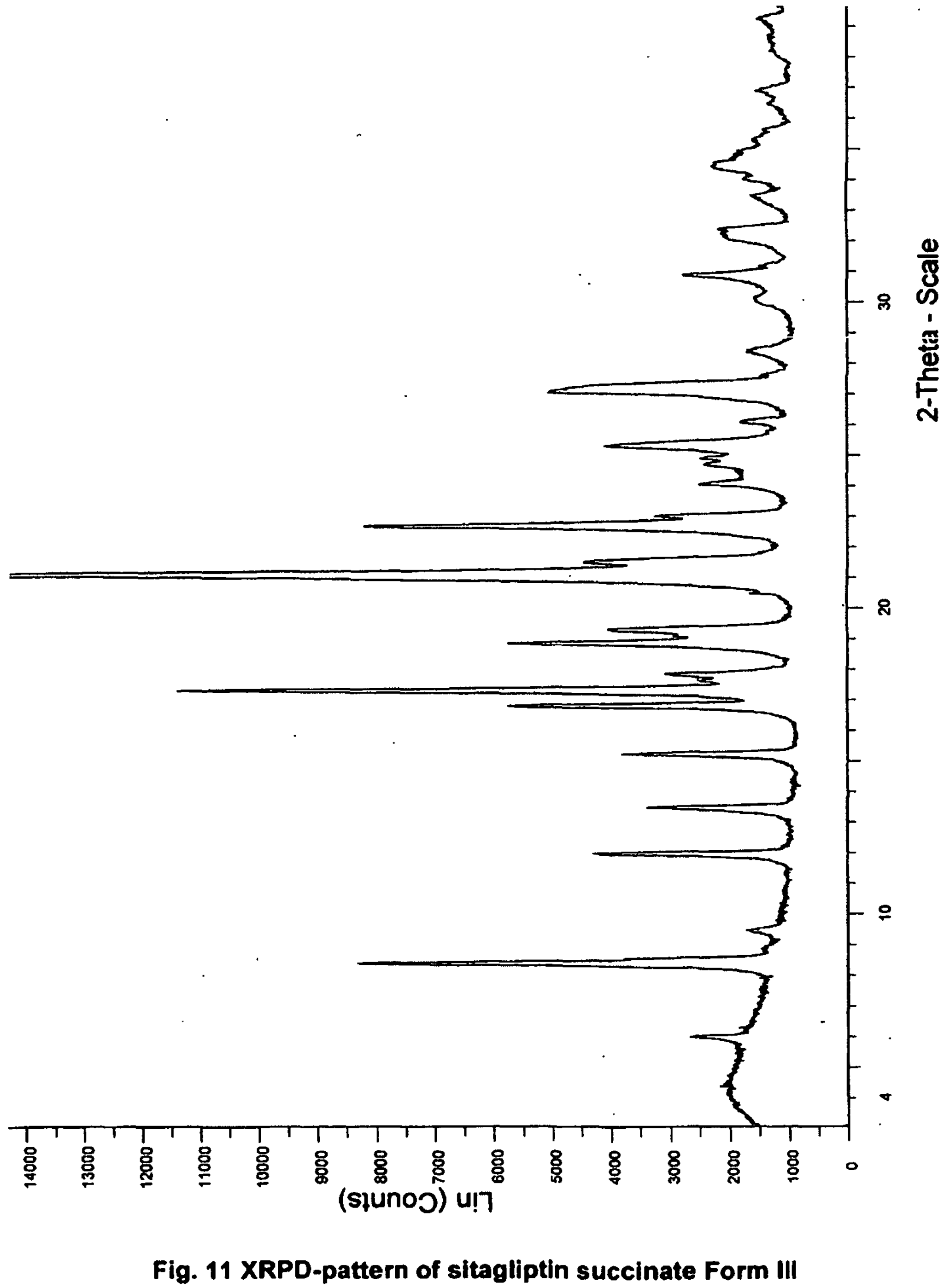


**Fig. 11 XRPD-pattern of sitagliptin succinate Form III**

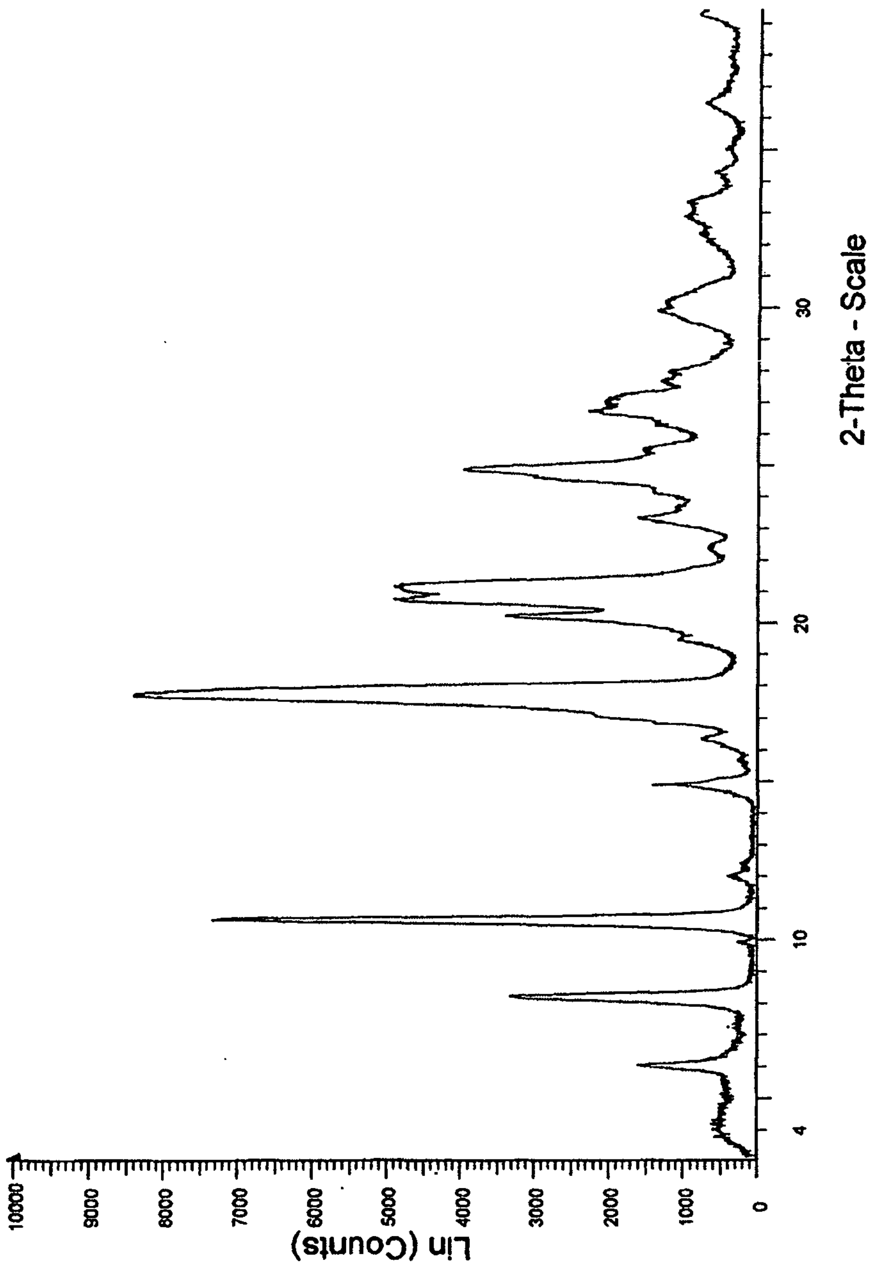


**Fig. 12 XRPD-pattern of sitagliptin lactate**

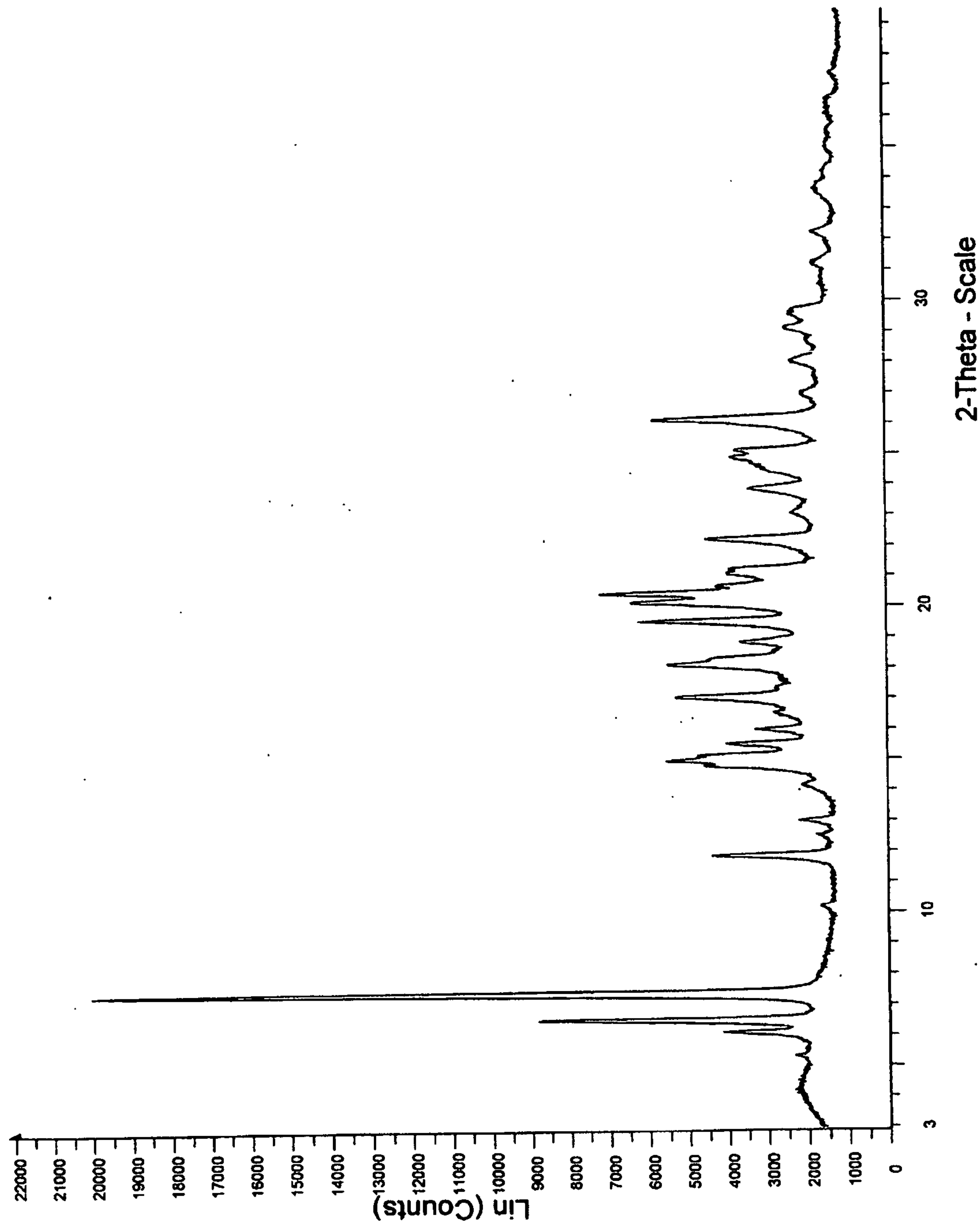


**Fig. 13 XRPD-pattern of sitagliptin glycolate**

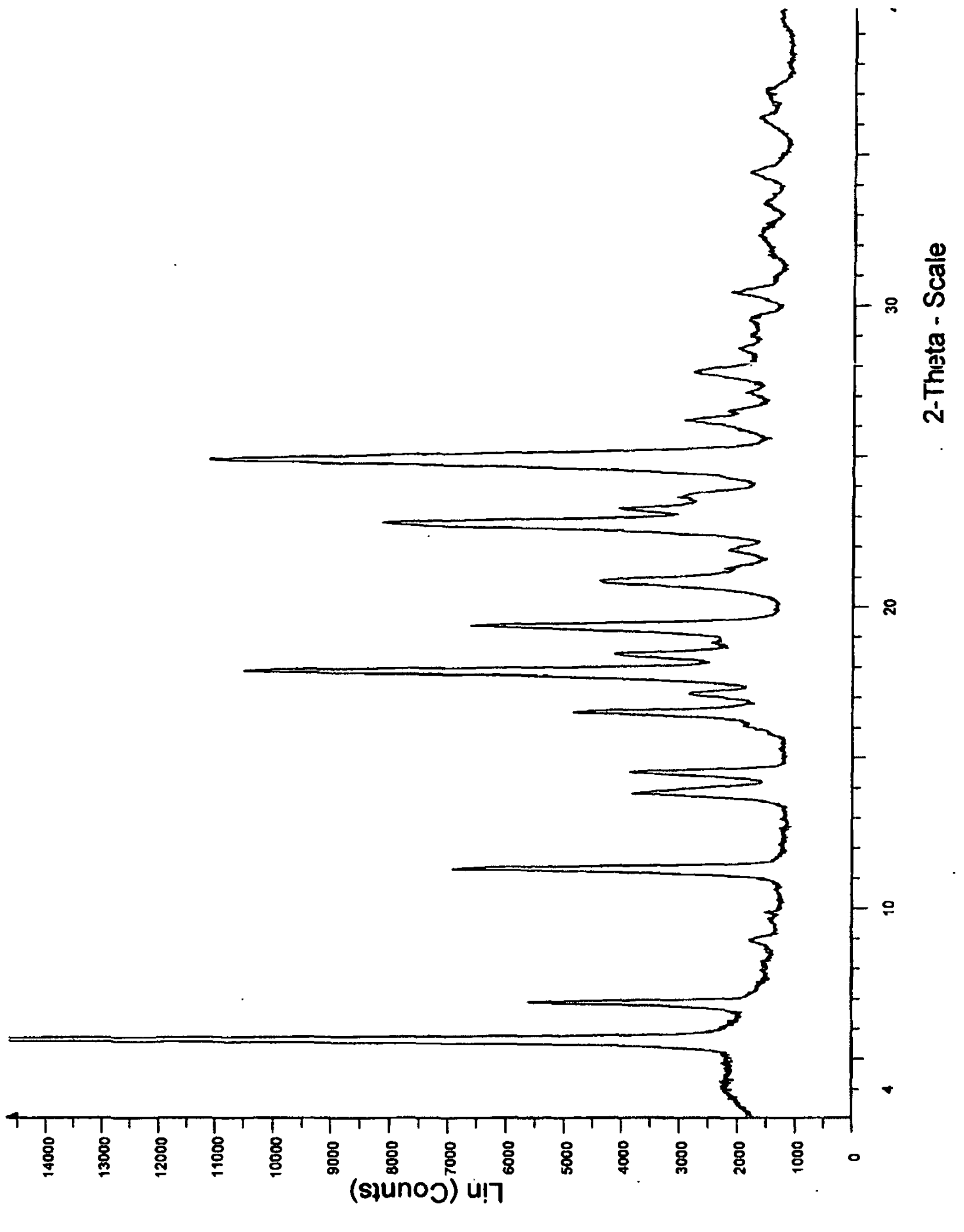


**Fig. 14 XRPD-pattern of sitagliptin maleate Form I**

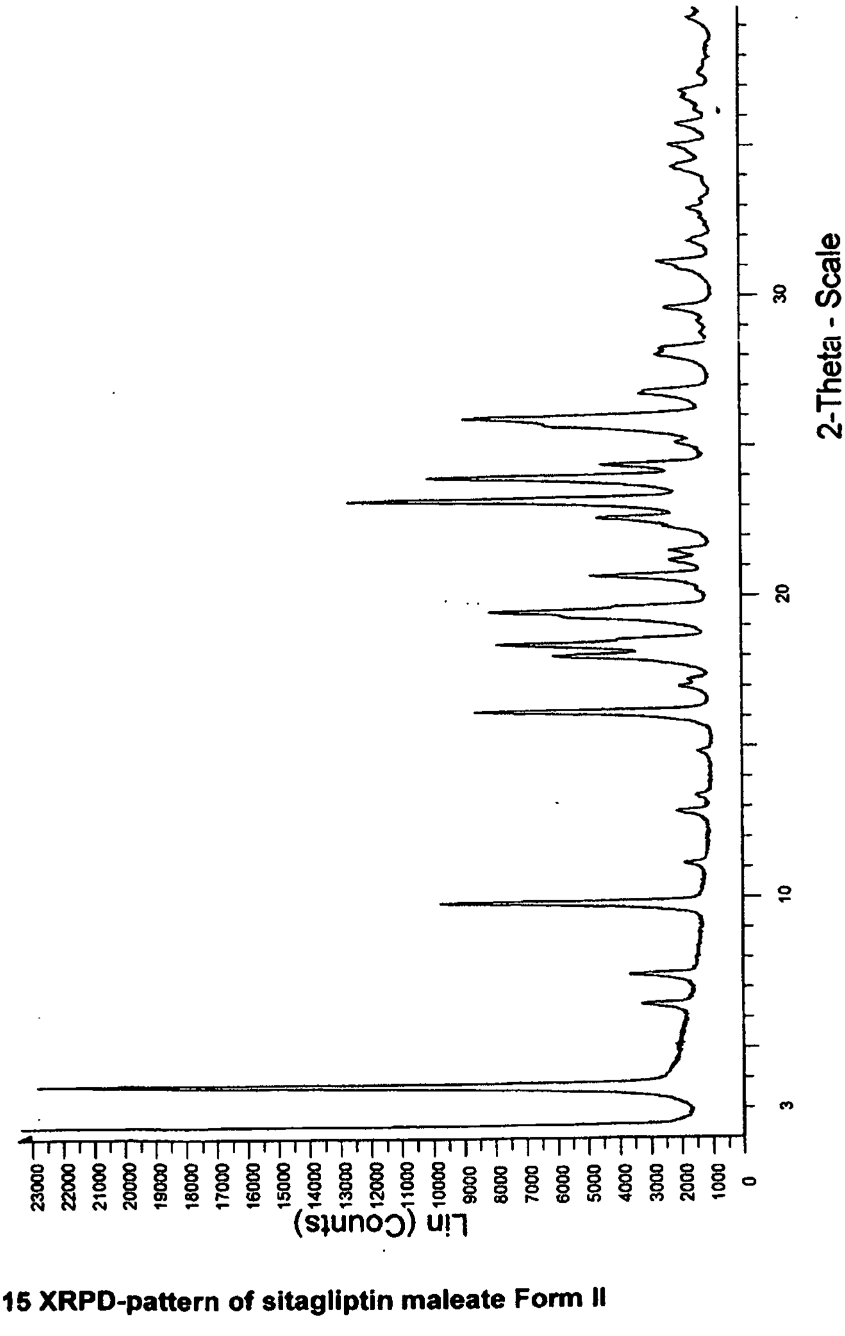


**Fig. 15 XRPD-pattern of sitagliptin maleate Form II**

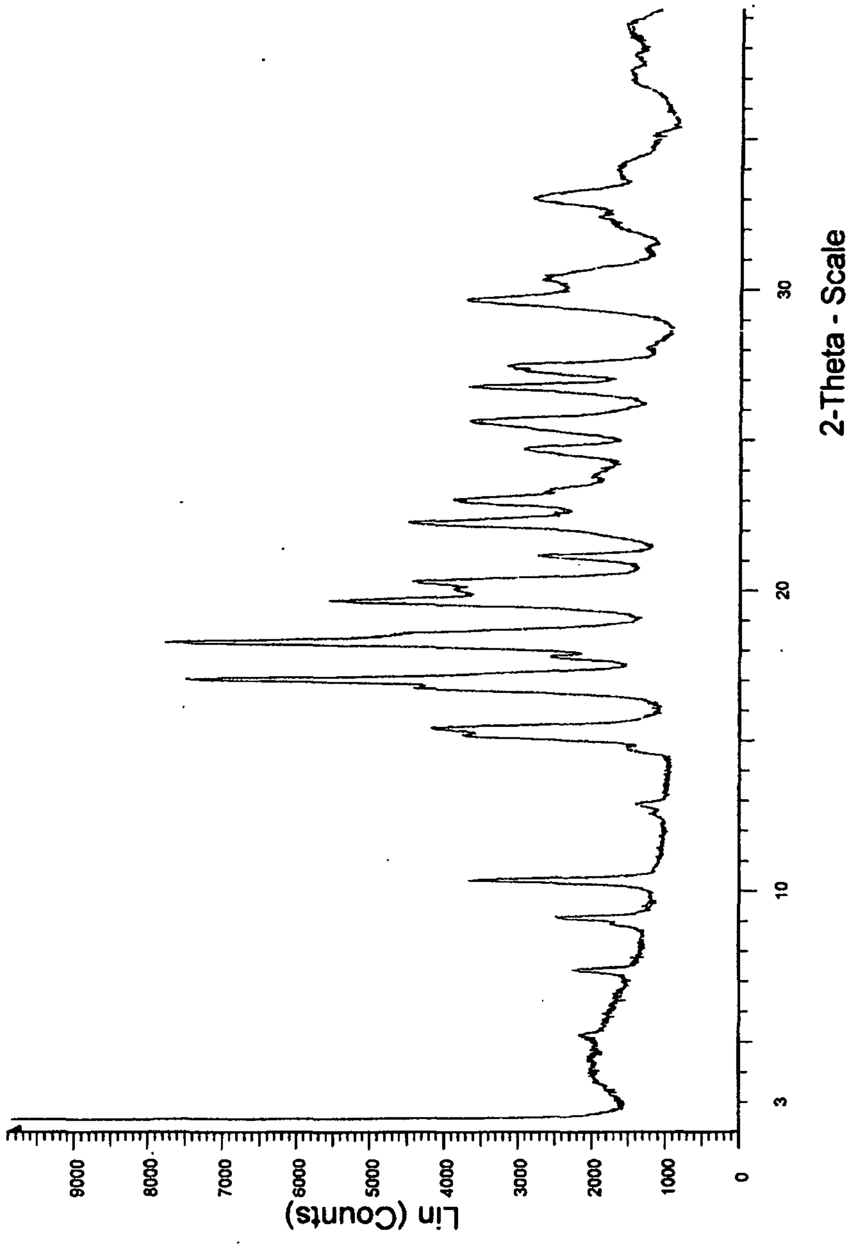


**Fig. 16 XRPD-pattern of sitagliptin citrate**

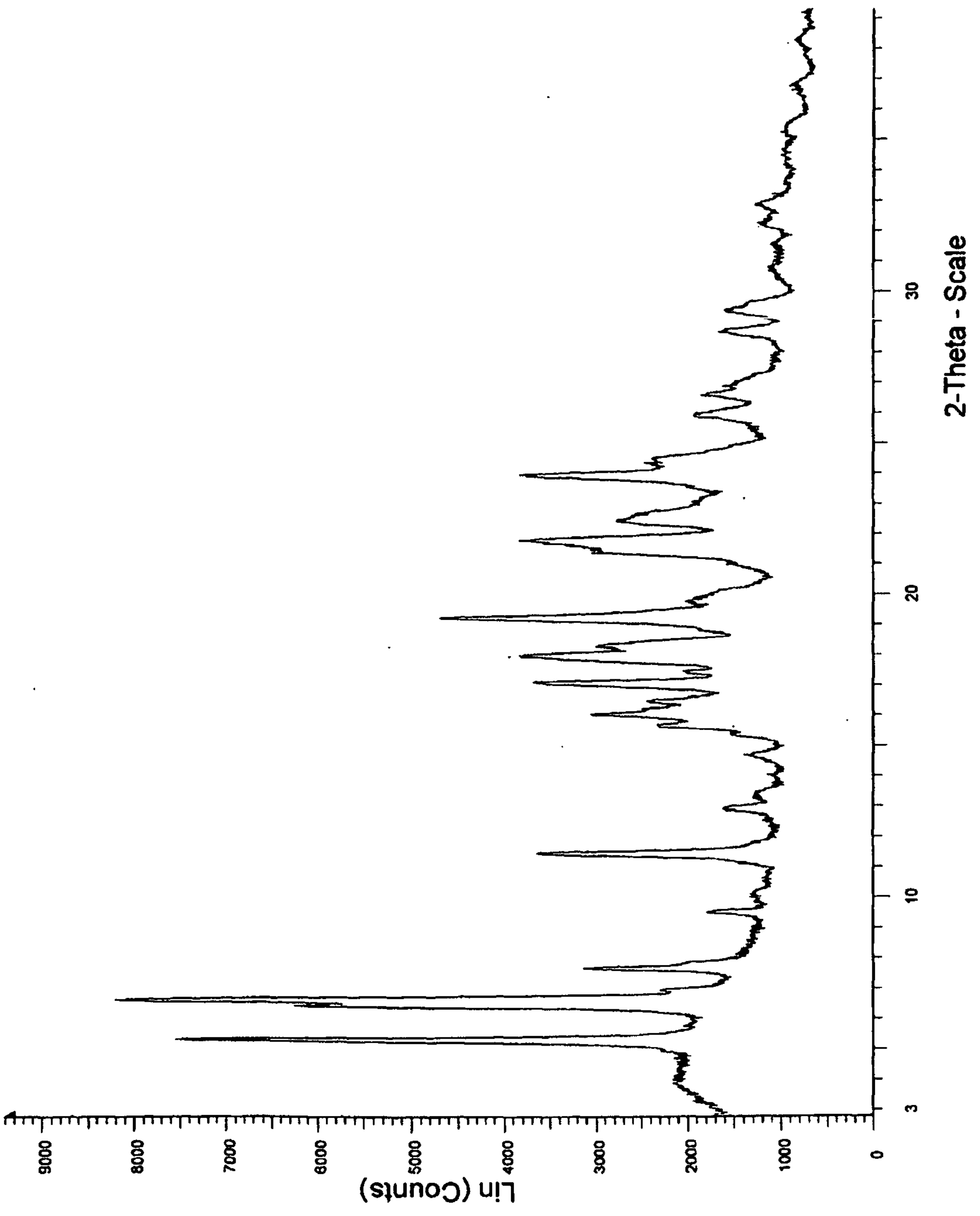


**Fig. 17 XRPD-pattern of sitagliptin mesylate Form I**

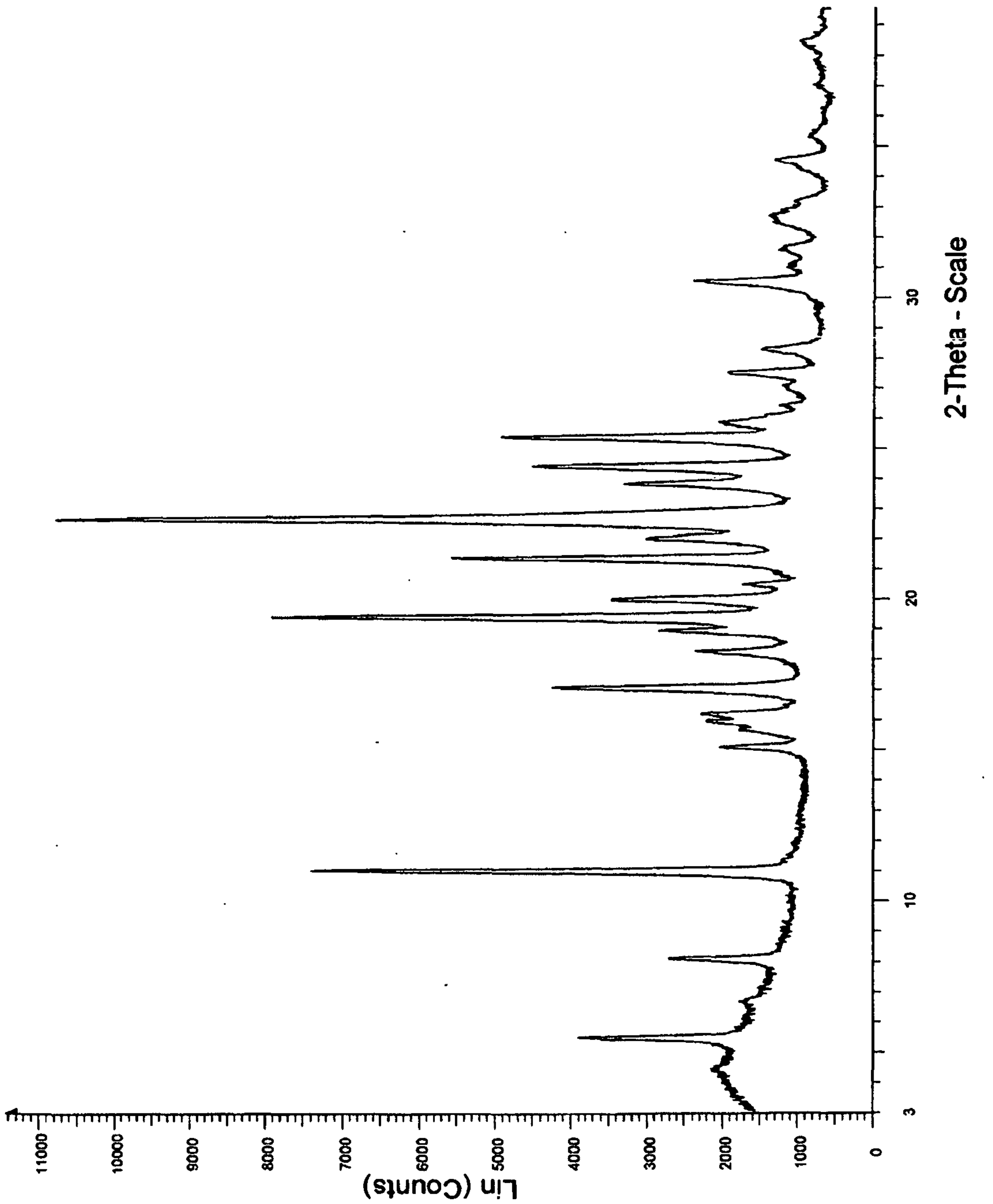


**Fig. 18 XRPD-pattern of sitagliptin mesylate Form II**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0896538 A
- WO 2003004498 A
- WO 03004498 A
- WO 2005072530 A
- WO 2005003135 A
- WO 2005020920 A
- WO 2005030127 A
- WO 2006033848 A

### Non-patent literature cited in the description


- **C. F. DEACON ; J. J. HOLST.** Dipeptidyl peptidase IV inhibition as an approach to the treatment and prevention of Type 2 diabetes: a historical perspective. *Biochem. Biophys. Res. Commun.,* 2000, vol. 294, 1-4
- **K. AUGUSTYNS et al.** Dipeptidyl peptidase IV inhibitors as new therapeutic agents for the treatment of Type 2 diabetes. *Expert. Opin. Ther. Patents,* 2003, vol. 13, 499-510
- **D. J. DRUCKER.** Therapeutic potential of dipeptidyl peptidase IV inhibitors for the treatment of Type 2 diabetes'', Expert Opin. Investig. *Drugs,* 2003, vol. 12, 87-100
- **M. A. NAUCK et al.** Incretins and Their Analogues as New Antidiabetic Drugs. *Drug News Perspect.,* 2003, vol. 16, 413-422
- **D. KIM et al.** *J. Med. Chem.,* 2005, vol. 48, 141-151